(19) Europäisches Patentamt
European Patent Office
Office européen des brevets

(11) **EP 3 547 014 A1**

(12) **EUROPEAN PATENT APPLICATION**
published in accordance with Art. 153(4) EPC

(43) Date of publication:
**02.10.2019 Bulletin 2019/40**

(51) Int Cl.:
**G02C 7/04** *(2006.01)*        **H04B 1/3827** *(2015.01)*

(21) Application number: **17873592.4**

(22) Date of filing: **14.11.2017**

(86) International application number:
**PCT/JP2017/040931**

(87) International publication number:
**WO 2018/096979 (31.05.2018 Gazette 2018/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **25.11.2016   JP 2016229109**
**26.12.2016   JP 2016251735**
**14.04.2017   JP 2017080269**

(71) Applicant: **Universal View Co., Ltd.**
**Tokyo 102-0093 (JP)**

(72) Inventors:
• **TAKAKI Yu**
**Tokyo 102-0093 (JP)**
• **SUZUKI Taro**
**Tokyo 102-0093 (JP)**
• **SUZUKI Yoshio**
**Tokyo 102-0093 (JP)**
• **NAGAI Kenji**
**Tokyo 102-0093 (JP)**
• **HASHIMOTO Masaru**
**Tokyo 102-0093 (JP)**

(74) Representative: **D Young & Co LLP**
**120 Holborn**
**London EC1N 2DY (GB)**

(54) **PINHOLE CONTACT LENS AND SMART CONTACT SYSTEM**

(57)     The invention enables quality of a pinhole image to be improved, any of myopia or hyperopia, astigmatism and presbyopia to be corrected and high functionality to attain. A pinhole contact lens 100 according to this invention includes a pinhole lens member 10 which contains a lens member 11 fittable onto a cornea of a person, a circular pinhole region 12 arranged approximately in a center of the lens member and forming a pinhole image on a retina of a wearer, a light-shielding region 13 provided on an outer peripheral side of the pinhole region 12 and shielding light, and a transparent region 14 provided on an outer peripheral side of the light-shielding region 13 and transmitting the light; and an electronic member 20 which is arranged on any one of the pinhole region 12, the transparent region 14 and the light-shielding region 13 and electrically performs an optical processing on any one of the light transmitted via the transparent region 14 and the light absorbed into the light-shielding region 13 on the basis of any control information, with respect to light passing through the pinhole region, to be applied on the pinhole image. Fig. 1.

【**FIG. 2**】

## Description

[TECHNICAL FIELD]

The present invention relates to a pinhole contact lens using with it being fitted onto a cornea and a smart contact system. Specifically, it relates to a smart contact lens in which an electronic member is arranged in at least any one of a pinhole region, a transparent region and a light-shielding region, and, electrically performs an optical processing on at least any one of the light transmitted via the transparent region and the light absorbed into the light-shielding region on the basis of any control information, with respect to light passing through the pinhole region, to be applied on the pinhole image, and a smart contact system using the same.

[BACKGROUND ARTS]

For an image of a subject visually recognized by a person, light of the subject incident from the cornea is refracted by the cornea and the crystalline lens, enters the vitreous body and is focused on the retina on the rear surface side of the vitreous body, and the imaged visual information is transmitted via the optic nerve to the cerebrum, so that the subject image is recognized visually. At this time, the subject light is focused on a position in front of or behind the retina, resulting in myopia or hyperopia. In addition, if the cornea or the crystalline lens is distorted, the subject light is not focused on the retina, resulting in astigmatism.

As means for correcting such myopia, hyperopia, astigmatism and the like, correction with glasses, correction with contact lenses and the like have been widely performed. Further, for correction of presbyopia, for example, bifocal glasses (bifocal lens) having a far sight part and a near sight part in one lens have been used.

On the other hand, pinhole contact lenses have been devised which can be worn on the eyes of a person. The pinhole contact lens has a lens member, and the lens member is provided with a pinhole region, a light-shielding region, and a transparent region. The pinhole region is arranged approximately at a central part of the lens member and has a circular shape. The light-shielding region is annularly provided on an outer peripheral side of the pinhole region, and the transparent region is annularly provided on an outer peripheral side thereof.

According to the pinhole contact lenses, when light is applied to the eyes of a person wearing the lenses, the light passes through the transparent region, whereas the light is shielded in the light-shielding region. Light at the center of the light limited by the light-shielding region is converged to and passes through the pinhole region. This causes formation, on the wearer's retina, of a pinhole image which provides a depth of focus. Here, the depth of focus refers to as "a range in the visual axis direction in which the subject looks clear in focus".

It is known that, when pinhole contact lenses of this type are used, an annular gray image (simply referred to as "a ring image") caused by light diffraction appears in the field of vision. In order to obtain brightness for reduction of this ring image, a contact lens having fine holes provided on an outer peripheral edge of a light-shielding region has been patented (see Patent Document 1).

Also, in recent years, a technology called a smart contact lens has been patented or disclosed. For example, an electronic contact lens has been disclosed in which an electronic structural element is mounted on a non-planar substrate for contact lens by applying a thin film manufacturing technology thereto (Patent Document 2).

Furthermore, an image display apparatus and a method configured so that a display unit such as a liquid crystal display device or an organic EL is mounted on a lens array that is fittable on the eyeball to form a virtual image by the display unit with respect to a real image from the external on the retina have been patented (Patent Document 3).

As a product specialized in ophthalmologic medical care, there has been disclosed a biosensor in which a wireless communication unit and a biological sensor are mounted on a resin lens, and the blood glucose level can be automatically measured using backscatter (backscatter wireless communication) (Patent Document 4).

Furthermore, a contact lens in which an optical communication unit and a light detector are mounted on a contact lens substrate to read out data in response to light modulation waves from outside has been patented (Patent Document 5).

In addition, an intraocular pressure monitoring device in which a pressure sensor is mounted on a contact lens and the corneal change is measured to monitor corneal deformation has been patented (Patent Document 6).

Furthermore, there has been disclosed an electrically-driven intraocular lens including an electrically-driven intraocular lens (IOL), an electrolytic capacitor and a piezoelectric film, wherein the charge generated by the piezoelectric film is stored in the capacitor according to the movement of the ciliary body and the power is used to drive the IOL so that the lens power can be controlled (Patent Document 7).

[DOCUMENTS FOR PRIOR ART]

[PATENT DOCUMENTS]

Patent Document 1: Japanese Patent No. 4828000

Patent Document 2: Japanese Patent Application Publication No. 2013-122591

Patent Document 3: Japanese Patent No. 4752309

Patent Document 4: US Patent No. 8608310

Patent Document 5: US Patent No. 9158133

Patent Document 6: Japanese Patent No. 5992424

Patent Document 7: Japanese Patent No. 5244092

[SUMMARY OF THE INVENTION]

[PROBLEMS TO BE SOLVED BY THE INVENTION]

By the way, in the pinhole contact lenses according to the prior art examples, there are the following problems.

i. In the pinhole contact lens described in Patent Document 1, fine holes for reducing the ring image and securing the brightness have been fixedly formed on the outer peripheral edge of the light-shielding region. The holes have a structure in which an optimum number of holes is found in advance and light based on the found number of holes is guided to a periphery of an image.

For this reason, uniform light has been focused on the periphery of the pinhole image through a bright place and a dark place (environment), and there has been a problem such that the pinhole image may disadvantageously be deteriorated.

ii. On the other hand, in each of the smart contact lenses (systems) described in Patent Documents 2 to 7, the contact lens have been provided with a display unit, a sensor and the like, and a real image by the contact lens has been formed on the retina while, with respect to the real image, a virtual image based on external information has been synthesized on the retina, and ophthalmology-specific data has been extracted.

Therefore, even though it has been possible to enhance the added value in an extremely general contact lens having no pinhole function, there has been a problem such that any added value cannot be disadvantageously enhanced in a contact lens to which the pinhole effect is applied, without any contrivance.

The present invention is made in view of the above problems and has an object to provide a pinhole contact lens which enables quality of a pinhole image to be improved, a correction of any of myopia or hyperopia, astigmatism and presbyopia to cope and high functionality to attain, and a smart contact system.

[MEANS FOR SOLVING THE PROBLEMS]

In order to solve the above problems, the pinhole contact lens according to the present invention includes: a pinhole lens member which contains a lens member fittable onto a cornea of a person, a pinhole region having a circular shape and arranged approximately in a center of the lens member, the pinhole region forming a pinhole image on a retina of a wearer, a light-shielding region provided on an outer peripheral side of the pinhole region and shielding light, and a transparent region provided on an outer peripheral side of the light-shielding region and transmitting the light; and an electronic member which is arranged on any one of the pinhole region, the transparent region and the light-shielding region and electrically performs an optical processing on any one of the light transmitted via the transparent region and the light absorbed into the light-shielding region on the basis of any control information, with respect to light passing through the pinhole region, to be applied on the pinhole image, as set forth in Claim 1. In the present invention, the phrase "approximately in a center" encompasses a central part of the lens member and a periphery of the central part.

According to the pinhole contact lens according to Claim 1, when the pinhole contact lens according to the present invention is put onto the cornea, the transparent region passes light while the light-shielding region shields the light. Light at the center which is limited by the light-shielding region is converged to and passes through the pinhole region. At this time, the electronic member can be used not only to dynamically correct the brightness around the pinhole image formed on the retina, but also to form a virtual image in conformity with the pinhole image itself.

A pinhole contact lens according to Claim 2 is the pinhole contact lens according to Claim 1, wherein the electronic member arranged on the pinhole lens member contains: a wireless communication unit which has an antenna element to transmit and receive the control information; a display unit which forms an image based on the control information around the pinhole image; a sensing unit which senses the light transmitted through the pinhole region; and a control unit which controls brightness around the pinhole image based on at least either one of sensing information from the sensing unit and the control information from the wireless communication unit.

A pinhole contact lens according to Claim 3 is the pinhole contact lens according to Claim 2, wherein the display unit contains a liquid crystal display element which is provided in a whole or a part of the pinhole region of the pinhole lens member and has a matrix shape.

A pinhole contact lens according to Claim 4 is the pinhole contact lens according to Claim 3, wherein the display unit forms an opening part for forming the pinhole image on the retina of the wearer in the pinhole region.

A pinhole contact lens according to Claim 5 is the pinhole contact lens according to Claim 4, wherein the display unit displays an alignment mark in the pinhole region.

A pinhole contact lens according to Claim 6 is the pinhole contact lens according to Claim 2, wherein the display unit contains a liquid crystal display element which is provided in a whole or a part of the light-shielding region of the pinhole lens member and has a matrix shape.

A pinhole contact lens according to Claim 7 is the pinhole contact lens according to Claim 6, wherein a plurality of fine holes is provided on an outer peripheral edge of the light-shielding region of the pinhole lens member, and wherein the control unit of the pinhole lens member performs liquid crystal switching control on the display unit arranged in the light-shielding region to control the display unit so as to select the holes.

A pinhole contact lens according to Claim 8 is the pinhole contact lens according to Claim 2, wherein the display unit contains a liquid crystal display element which is provided in a whole or a part of the transparent region of the pinhole lens member and has a matrix shape.

A pinhole contact lens according to Claim 9 is the pinhole contact lens according to Claim 3 or 8, wherein the liquid crystal display element includes a dot color film member of three colors of red, blue and green.

A pinhole contact lens according to Claim 10 is the pinhole contact lens according to Claim 2, wherein the pinhole lens member includes a power supply unit which drives the wireless communication unit, the sensing unit, the display unit and the control unit, and wherein the power supply unit includes a power storage element having any one of a wireless induction feeding system, an optical modulation wave feeding system, and a solar power feeding system.

A pinhole contact lens according to Claim 11 is the pinhole contact lens according to Claim 2, wherein the control unit includes a microprocess element which is provided in the light-shielding region of the pinhole lens member, and connected to the wireless communication unit, the display unit and the sensing unit to control input/output of the wireless communication unit, the sensing unit, and the display unit based on the control information.

A pinhole contact lens according to Claim 12 is the pinhole contact lens according to Claim 1, wherein the electronic member contains a self-emitting element arranged at least on a cornea-wearing side of the light-shielding region of the pinhole lens member, and performs display control on light from the self-emitting element based on control information to be applied on the pinhole image.

A pinhole contact lens according to Claim 13 is the pinhole contact lens according to Claim 12, wherein the electronic member contains: a wireless communication unit which includes an antenna element to transmit and receive the control information; a display unit which includes a red light emitting element, a blue light emitting element, and a green light emitting element, and forms an image based on the control information around the pinhole image; and a control unit which controls the display unit to form an image based on the control information from the wireless communication unit around the pinhole image.

A pinhole contact lens according to Claim 14 is the pinhole contact lens according to Claim 2 or 13, wherein the wireless communication unit contains an antenna element which is provided along an outer peripheral edge of the transparent region of the pinhole lens member and has a loop shape.

A pinhole contact lens according to Claim 15 is the pinhole contact lens according to Claim 13, wherein the control unit contains a microprocess element which is provided in the light-shielding region of the pinhole lens member, and connected to the wireless communication unit and the display unit to control input/output of the wireless communication unit and the display unit based on the control information.

A pinhole contact lens according to Claim 16 is the pinhole contact lens according to Claim 13, wherein the pinhole lens member includes a power supply unit which drives the wireless communication unit, the display unit, and the control unit, and wherein the power supply unit includes a power storage element having any one of a wireless induction feeding system, an optical modulation wave feeding system and a solar power feeding system.

A pinhole contact lens according to Claim 17 is the pinhole contact lens according to Claim 16, wherein the electronic member in at least a part of the wireless communication unit, the display unit, the control unit, and the power supply unit is mounted on a C-shaped base main body which is wearable in an eyelid of a person.

A pinhole contact lens according to Claim 18 is the pinhole contact lens according to Claim 1 or 12, wherein the pinhole lens member is provided with an imaging element.

A pinhole contact lens according to Claim 19 is the pinhole contact lens according to Claim 1 or 12, wherein the pinhole lens member is provided with a storage element.

A pinhole contact lens according to Claim 20 is the pinhole contact lens according to Claim 1 or 12, wherein the pinhole lens member is provided with a pressure sensing element.

A pinhole contact lens according to Claim 21 is the pinhole contact lens according to Claim 1 or 12, wherein the lens member contains a top surface side lens member and a rear surface side lens member, and wherein the electronic member has a sandwich structure in which the electronic member is sandwiched by the top surface side lens member and the rear surface side lens member.

A pinhole contact lens according to Claim 22 is the pinhole contact lens according to Claim 1 or 12, wherein the pinhole lens member is composed of at least either one of a soft contact lens and a hard contact lens.

A smart contact system according to Claim 23 includes: the pinhole contact lens according to any one of Claims 1 to 22; an external device capable of data communication with the pinhole contact lens; and a smartphone capable of data communication with the external device.

[EFFECTS OF THE INVENTION]

According to the pinhole contact lens according to the present invention, it is possible to dynamically correct the brightness around the pinhole image formed on the retina, so an annular gray image (ring image) caused by light diffraction can be reduced.

Further, according to the present invention, it becomes possible to selectively control a plurality of fine holes pierced on the outer peripheral edge of the light-shielding region. So, the brightness around the pinhole image can be precisely controlled in response to the bright environment and the dark environment. In addition, it is possible to form a real image by the pinhole while, with respect to the real image, synthesizing a virtual image based on control information on the retina.

As a result, it is possible to make the brain of the contact lens wearer recognize in real time an image or the like based on the control information while maintaining the functions of the conventional fixed hole number type pinhole contact lenses. Therefore, it is possible to provide a smart contact lens which enables quality of a pinhole image to be improved, a correction of all of myopia or hyperopia, astigmatism and presbyopia to cope by utilizing the original depth of focus provided by the pinhole and high functionality to attain.

The smart contact system according to the present invention includes any of the pinhole contact lenses according to the present invention, thereby making it possible to provide a remote diagnosis treatment comprehensive system using a smart contact lens in which the focal of depth provided by the pinhole is utilized, correction of all of myopia or hyperopia, astigmatism and presbyopia is dealt with, and high functionality such as intraocular pressure, blood sugar and light detection functions are implemented.

[BRIEF DESCRIPTION OF THE DRAWINGS]

FIG. 1 is a perspective view of a pinhole contact lens according to each embodiment of the present invention showing a configuration example thereof.

FIG. 2 is a plan view of a pinhole contact lens 100 according to a first embodiment showing a configuration example thereof.

FIG. 3 is a front view of the pinhole contact lens 100 showing a wearing example thereof.

FIG. 4 is a side view and a front view of an eyeball at the time of wearing according to a functional example of the pinhole contact lens 100.

FIG. 5 is a block diagram showing a configuration example of a control system according to each embodiment.

FIG. 6 is a block diagram showing a configuration example of a smart contact system 900 according to each embodiment.

FIG. 7 is an exploded perspective view of the pinhole contact lens 100 showing a formation example thereof.

FIG. 8 is a plan view of a pinhole contact lens 200 according to a second embodiment showing a configuration example thereof.

FIG. 9 is a flowchart of a brightness correction mode in the pinhole contact lens 200.

FIG. 10 is a side view and a front view of an eyeball, showing a functional example of a pinhole contact lens 300 according to a third embodiment.

FIG. 11 is a flowchart of an alignment assistance mode in the pinhole contact lens 300.

FIG. 12 is a plan view of a pinhole contact lens 400 according to a fourth embodiment showing a configuration example thereof.

FIG. 13 is a flowchart of a first image synthesis mode in the pinhole contact lens 400.

FIG. 14 is a plan view of a pinhole contact lens 500 according to a fifth embodiment showing a configuration example thereof.

FIG. 15 is a side view and a front view of an eyeball, showing a functional example of the pinhole contact lens 500.

FIG. 16 is a flowchart of a second image synthesis mode in the pinhole contact lens 500.

FIG. 17A is a front view of a pinhole contact lens 600 according to a sixth embodiment at the time of wearing of a base main body 38, showing a configuration example thereof.

FIG. 17B is a front view showing a putting example of a drug containing unit 33 and an electronic member 20.

FIG. 18 is a flowchart of an intraocular pressure sensing mode in the pinhole contact lens 600.

FIG. 19 is a plan view of a pinhole contact lens 700 according to a seventh embodiment showing a configuration example thereof.

FIG. 20 is a flowchart of an imaging mode in the pinhole contact lens 700.

[EMBODIMENT FOR CARRYING OUT THE INVENTION]

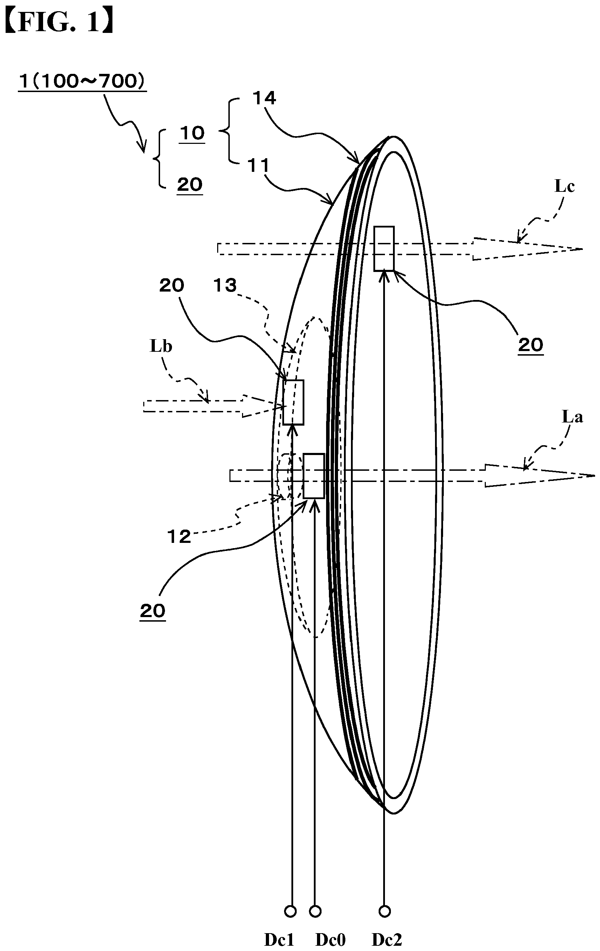

The following will describe the best mode for carrying out the present invention (hereinafter referred to as embodiment) with reference to FIGS. 1 to 20.

<First Embodiment>

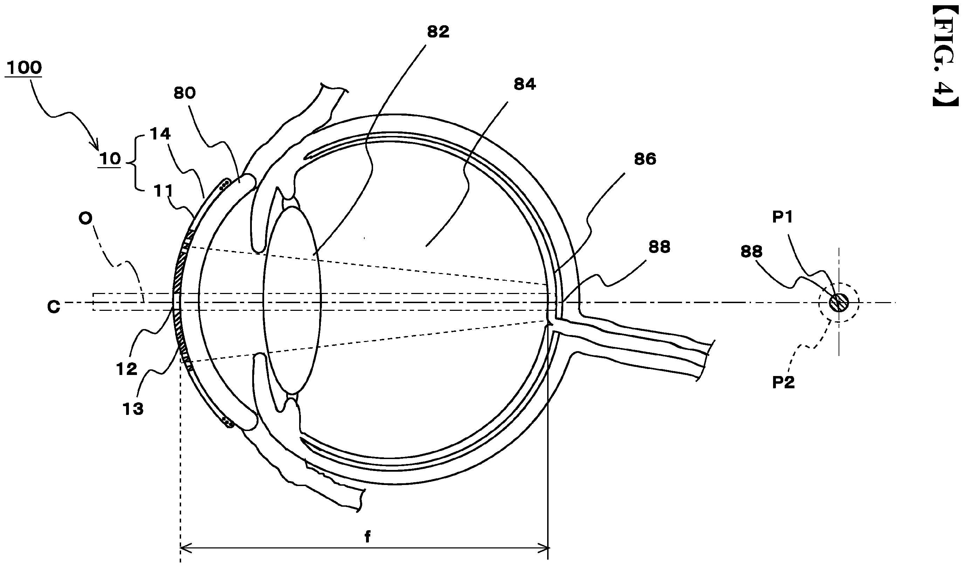

A pinhole contact lens 1 shown in FIG. 1 is a smart contact lens, and represents a configuration example of any of the pinhole contact lenses 100 to 700 of each embodiment according to the present invention which will be described later. Hereinafter, the configuration example of the pinhole contact lens 1 according to each embodiment will be described using the pinhole contact lens 100 of the first embodiment. In the pinhole contact lenses 200 to 700 of the second to seventh embodiments, the same components as those of the pinhole contact lens 100 of the first embodiment are denoted by the same codes, and the detailed descriptions thereof will be omitted. The pinhole contact lens 100 has a pinhole lens member 10 fittable on the cornea of a person and an electronic member 20 in FIG. 1. The pinhole lens member 10 has a lens member 11. A pinhole region 12 is arranged approximately at the center of the lens member 11, and a beam of light La passing through the pinhole region 12 comes to form a pinhole image on a wearer's retina 86 (see FIG. 4A). Note that the phrase "approximately at the center of the lens member 11" encompasses a central part of the lens member 11 and a periphery of the central part.

A light-shielding region 13 is provided on the outer peripheral side of the pinhole region 12 to shield light Lb. The pinhole region 12 is preferably configured to be formed in the shape of a perfect circle for formation of the pinhole image with good reproducibility. A transparent region 14 is provided on the outer peripheral side of the light-shielding region 13. The transparent region 14 allows light Lc to pass therethrough.

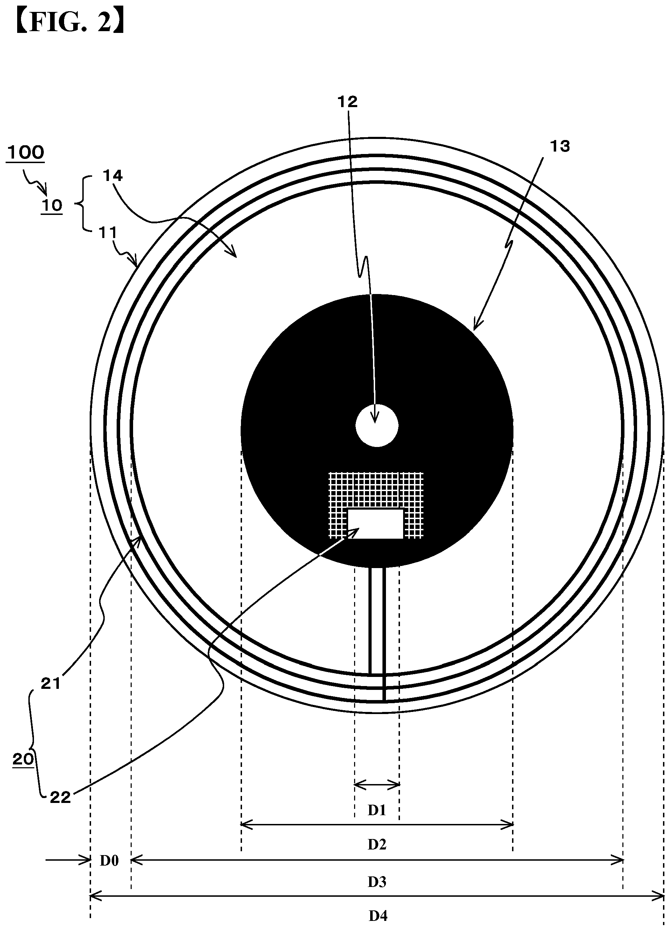

The electronic member 20 including an antenna element 21 (see FIG. 2) is arranged in any one of the pinhole region 12, the light-shielding region 13 and the transparent region 14. The electronic member 20 functions to electrically perform optical processing on at least either one of the light Lb absorbed by the light-shielding region 13 and the light Lc passing through the transparent region 14, with respect to the light La passing through the pinhole region 12, based on control information Dc1 and Dc2 to be applied on the pinhole image. Furthermore, optical processing (display control) is performed on the light La passing through the pinhole region 12 based on control information Dc0.

The pinhole lens member 10 shown in FIG. 2 is composed of at least either one of a soft contact lens and a hard contact lens, and has the lens member 11 having a diameter D4. In the lens member 11, the pinhole region 12 having a diameter D1, the light-shielding region 13 having a diameter D2 and the transparent region 14 having a diameter D3 are defined concentrically.

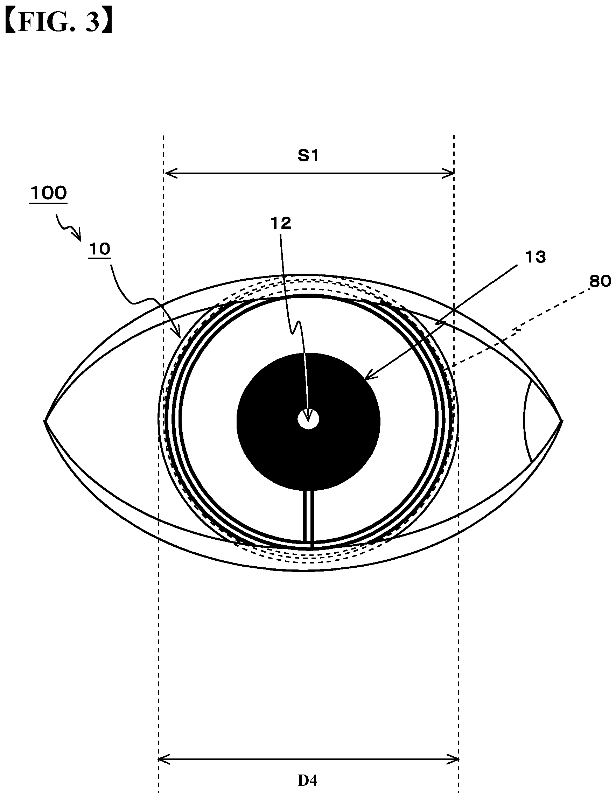

The diameter D4 of the lens member 11 is selected to be larger than the diameter D2 of the light-shielding region 13 and to be, at least, larger than a diameter S1 (for example, 12 mm) of a cornea 80 as shown in FIG. 3, and is selected, for example, to be about 14 mm. This is because, when the diameter D4 of the lens member 11 is set to be smaller than the cornea diameter, the lens member 11 may move greatly on the cornea 80, and the center of the cornea 80 and the center C of the light-shielding region 13 (see FIGS. 4A and 4B) are significantly shifted so that stable vision cannot be secured. In addition, this is because, when the diameter D4 of the lens member 11 exceeds 14 mm, it becomes difficult to put the lens member 11 onto the cornea 80.

The lens member 11 is a circular member, in plane view, having a curved surface (curvature) along the surface shape of the cornea 80, and is made of a light-transmitting material that transmits incident light. As the material for the lens member 11, hydroxyethyl methacrylate (HEMA), N-vinyl pyrrolidone (N-VP), dimethyl acrylamide (DMAA), glycerol methacrylate (GMA), silicone hydrogel (SH) and the like, which are used in water-containing soft contact lenses, are suitably used. In addition, as the material for the lens member 11, silicone rubber, butyl acrylate and dimethylsiloxane lenses, which are used in non-water-containing soft contact lenses, water-containing soft contact lenses, etc., are also suitably used. A colored material such as a blue- or red-colored material may be used in addition to a transparent material, as long as the material can transmit light.

The lens member 11 is not limited to a soft contact lens, and may be composed of a hard contact lens. For example, as the material for the lens member 11, a so-called oxygen-permeable siloxanyl methacrylate (SMA)-based hard contact lens which transmits some oxygen, or an oxygen impermeable methyl methacrylate (MMA) hard contact lens is used. Of course, the lens member 11 may be a silicone hydrogel lens having the merits of both the soft contact lens and the hard contact lens in combination.

The lens member 11 is also used as a replaceable or disposable soft contact lens. In the case of a person with mild myopia, hyperopia, or astigmatism, it is considered that both far vision and near vision can be obtained without adding the power. In other cases, it is also possible to add a power according to the user's myopia or hyperopia state to the lens member 11. As shown in FIG. 2, the lens member 11 located at the peripheral part of the light-shielding region 13 has the transparent region 14 for securing a peripheral vision by taking incident light into the cornea 80. The outer peripheral edge part of the transparent region 14 functions as an arrangement region for the antenna element 21 constituting the electronic member 20. In FIG. 2, assuming that the winding start diameter of the antenna element 21 is D3, the width of an arrangement region D0 is (D4-D3)/2.

The pinhole region 12 constitutes an example of the opening part, and, as shown in FIG. 2 and FIGS. 4A and 4B, is pierced at a center C of the light-shielding region 13 (lens member 11) and at a position including an optical axis (eye axis) O connecting the center of the cornea 80 and a central fovea 88 of the retina 86. The hole shape of the pinhole region 12 is preferably a perfect circle or a true circle in order to prevent diffraction of incident light. Also, the pinhole region 12 has a function of limiting a light flux of incident light entering the cornea 80 to a fixed amount to form a pinhole image P1 on the retina 86, and provides the effect corresponding to the addition power of a multifocal lens according to the size of the diameter D1 of the pinhole region 12. In FIG. 4B, a brightness image P2 is a brightness image around the pinhole image.

The diameter D1 of the pinhole region 12 is selected, for example, in the range of 1.0 mm to 1.6 mm. It has been recognized that, when the diameter D1 of the pinhole region 12 is changed in the range of 1.0 mm to 1.6 mm, a near vision corresponding to an addition power of approximately 1.00 to 3.00D can be obtained depending on the change of the size of the diameter. In addition, when the diameter D1 of the pinhole region 12 is selected to be less than 1.0 mm or more than 1.6 mm, it was not possible to obtain an optimal near vision. The reason for this is considered to be that, as shown in FIG. 4A, even in the case of an unadjusted lens, the depth of focus is deepened by changing the light flux. In the embodiments which will be described later, the electronic member 20 is mounted in the pinhole region 12 having the diameter D1 and electrically performs optical processing.

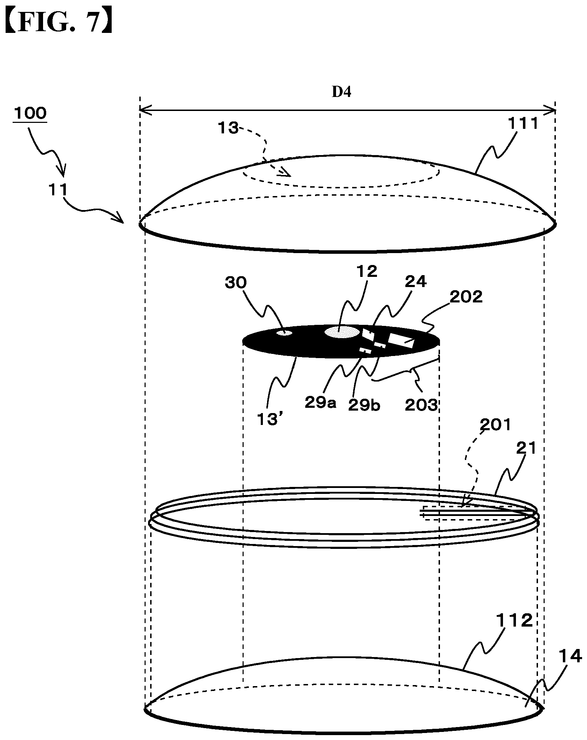

The light-shielding region 13 is a circular member, in plane view, having a curved surface along the surface shape of the cornea 80 (see FIG. 4A), and is composed of a light-shielding member 13' that shields the light Lb incident on the cornea 80 (see FIG. 7). The light-shielding member 13' has a black color, and, as the material therefor, a material whose safety has already been established and which is used in an actual soft contact lens with iris is suitably used. For example, an azo colorant (red) or a phthalocyanine colorant (blue) can be used. The light-shielding region 13 is preferably designed to have a certain thickness in order to provide the linearity of the light La passing through the pinhole region 12. On the contrary, it is preferable to make the transparent region 14 as thin as possible in order to prevent interception of light beams. A squid ink may be used for the light-shielding member 13'. The squid ink is used because it is friendly to the ecological system.

The diameter D2 of the light-shielding region 13 is selected in the range of, for example, 4.0 mm to 9.0 mm, in consideration of the pupil diameter at the time of mydriasis (the size of the pupil in the dark) which changes according to the age. The reason for this is as follows. When the diameter D2 of the light-shielding region 13 is less than 4.0 mm, the area of the transparent region 14 (see FIG. 2), which will be described later, around the light-shielding region 13 becomes large, and the peripheral vision becomes wide. However, if the pupil diameter is larger than the diameter of the light-shielding region 13 at night or the like, halo and glare may be caused since ambient light other than the light in the light-shielding region 13 enters the center of the retina 86 in some cases.

Further, when the diameter D2 of the light-shielding region 13 is more than 9.0 mm, the area of the transparent region 14 transmitting the incident light of the lens member 11 becomes small, so that the peripheral vision cannot be secured. That is, the same field of vision (visual sense) as that of the naked eye cannot be secured. The pinhole contact lens 100 according to the present invention can cope with correction of all of light myopia or hyperopia, astigmatism, and presbyopia by utilizing the depth of focus provided by the pinhole region 12 pierced at the center of the light-shielding region 13.

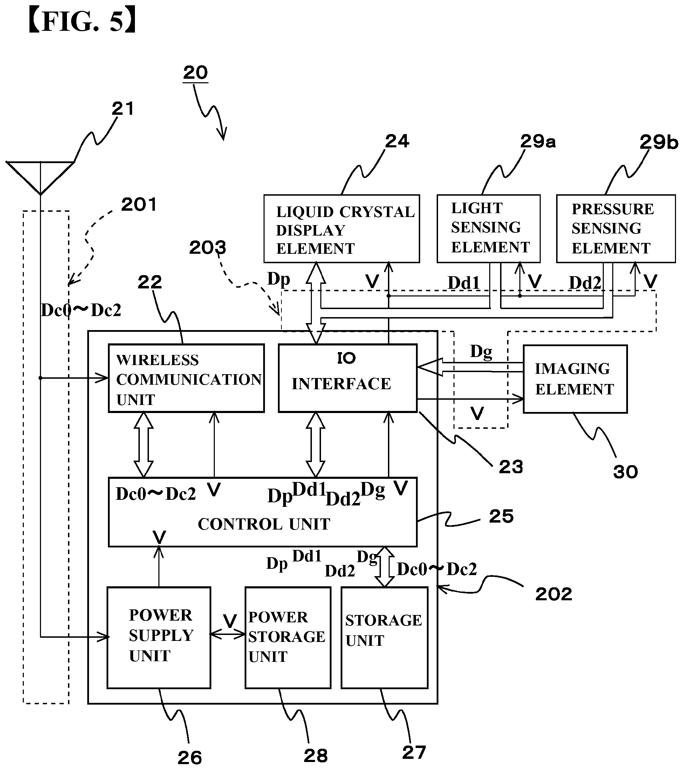

The transparent region 14 is composed of the lens member 11. The antenna element 21 is provided at the outer peripheral edge part of the transparent region 14 so as to transmit and receive the control information Dc0 to Dc2 to and from an external device 90 (see FIGS. 5 to 7).

For example, as shown in FIG. 5, the electronic member 20 arranged in the pinhole lens member 10 is composed of a first printed wiring part 201, a one-chip operation element 202, a second printed wiring part 203, a liquid crystal display element 24, a light sensing element 29a and a pressure sensing element 29b. These wiring parts and elements may be configured by a flexible printed circuit (FPC). The FPC is flexible, can be deformed repeatedly by a weak force, and has a characteristic of maintaining its electrical characteristics even when deformed.

The antenna element 21 is connected to one end of the first printed wiring part 201, and the one-chip operation element 202 is connected to the other end thereof. The size of the one-chip operation element 202 is, for example, about 2 mm × 2 mm. The one-chip operation element 202 is composed of a semiconductor integrated circuit. As shown in FIG. 2, the antenna element 21 has a loop shape and is provided along the outer peripheral edge of the transparent region 14.

The one-chip operation element 202 is provided, for example, in the light-shielding region 13 and has a wireless communication unit 22, an IO interface 23, a control unit 25, a power supply unit 26, a storage unit 27 and a power storage unit 28, which are integrated in one chip. As the one-chip operation element 202, one that can operate at about several mW is used. Of course, the one-chip operation element 202 may be provided in the transparent region 14, but is preferably provided in the light-shielding region 13 in order to reduce the wiring density of the second printed wiring part 203.

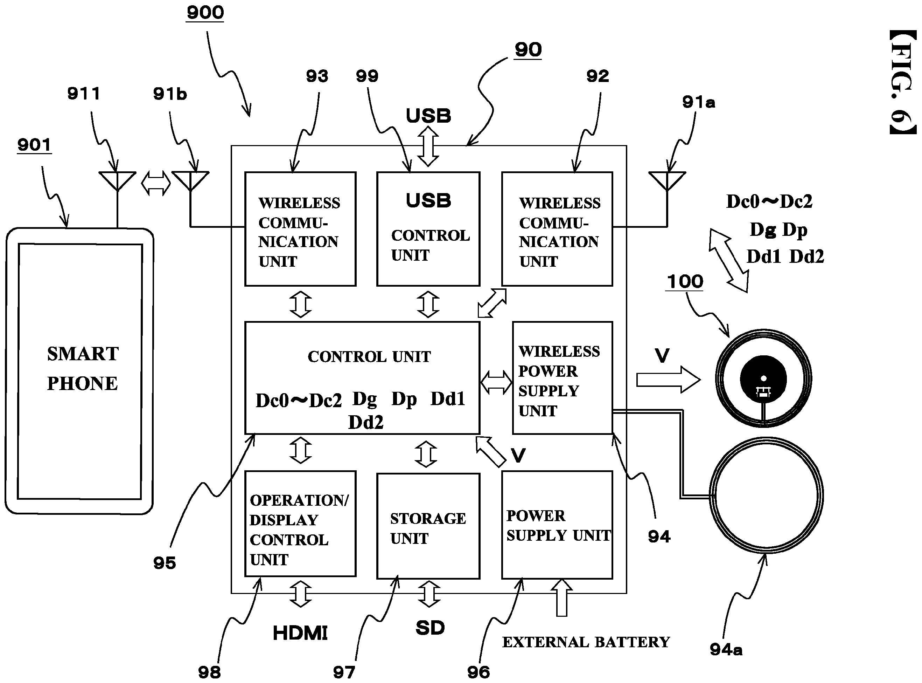

The antenna element 21 is connected to the wireless communication unit 22, transmits the control information Dc0 to Dc2 to the wireless communication unit 22 to the external device 90, and receives the control information Dc0 to Dc2 from the external device 90 (see FIG. 6). The wireless communication unit 22 transmits and receives the control information Dc0 to Dc2 according to, for example, a short-range wireless system (Bluetooth) (registered trademark). The external device 90 is a dedicated terminal device and can communicate via an antenna element 911 of a smartphone 901 or the like as shown in FIG. 6. As the external device 90, one having at least an operation setting function, a control information setting function, an information writing/reading function, and a power supplying function is used. The power loss of the received power which can be received by the wireless communication unit 22 can be reduced by improving impedance matching between the antenna element 21 and the wireless communication unit 22, reducing the antenna loss, and designing the best rectifier circuit.

The control unit 25 is connected to the wireless communication unit 22, and the IO interface 23 is connected to the control unit 25. The liquid crystal display element 24 as an example of the display unit, the light sensing element 29a and pressure sensing element 29b as examples of the sensing unit, and the like are connected to the IO interface 23. The IO interface 23 assists the control unit 25 in an auxiliary manner, and controls input/output of various kinds of information. The liquid crystal display element 24 is assumed to be handled as a transparent display and to utilize the shutter function of liquid crystal. In the display unit, a surface-emitting element (self-emitting array) in which laser diodes are spread may be used in some cases. The liquid crystal display element 24 may be arranged to straddle the light-shielding region and the transparent region. A boundary circular line between these regions can be expressed by liquid crystal. The boundary circular line refers to as a line of circular boundary which divides the pinhole region 12 and the light-shielding region 13 or the light-shielding region 13 and the transparent region 14 from each other.

The liquid crystal display element 24 has a matrix shape and is provided in the whole or a part of the pinhole region 12 in the embodiments which will be described later. The liquid crystal display element 24 has a liquid crystal switching function for turning on/off the transmitted light. Of course, in addition to the above-described function, one having a dot color film member of three colors of red, blue and green (hereinafter referred to as RGB colors) is also used as the liquid crystal display element 24 to perform color display in RGB colors. Of course, the synthesis of three-color pixels provides a sunglasses effect of shielding light having a specific wavelength.

The liquid crystal display element 24 uses a simple matrix (SM) driving system or an active matrix (AM) driving system. The SM driving system is referred to also as a passive matrix driving system (PM type), in which a voltage is applied to a pixel or a sub pixel at an intersection of an X electrode wire and a Y electrode wire to drive liquid crystal. In the AM type driving system, a thin film transistor (TFT) is used, and, in addition to the X and Y electrode wires and storage capacitors of the simple matrix, an active element is provided for each pixel.

The light sensing element 29a is provided, for example, in the pinhole region 12, senses the light La transmitted through the pinhole region 12, and outputs light sensing information Dd1 to the control unit 25. The light sensing element 29a is not limited to be provided in the pinhole region 12, and it may be provided in the transparent region 14. It may sense the light passing through the transparent region 14. The pressure sensing element 29b (piezoelectric sensor) is provided in the light-shielding region 13 of the pinhole lens member 10. The pressure sensing element 29b is connected to the control unit 25, senses the pressure (intraocular pressure) of the cornea 80, and outputs intraocular pressure sensing information Dd2 to the control unit 25. The pressure sensing element 29b is not limited to be provided in the light-shielding region 13, and it may be provided in the transparent region 14. It may sense the pressure applied to the transparent region 14.

The imaging element 30 is provided, for example, in the light-shielding region 13 of the pinhole lens member 10. The imaging element 30 is connected to the IO interface 23, images an object, and outputs imaging information Dg to the control unit 25. A solid-state imaging element array including a lens system is used in the imaging element 30, and the light passing through the lens system is photoelectrically converted. For example, a charge coupled device (CCD) sensor array or a complementary metal oxide semiconductor (CMOS) sensor array is used as the solid-state imaging element array. The imaging element 30 is not an essential component, and may be mounted in accordance with the design request.

The control unit 25 constitutes an example of the electronic member 20, and includes a microprocess element having a computer function. For example, a microprocess element which consumes a power of about several mW can be used. The one-chip operation element 202 mounted with the microprocess element is provided in the light-shielding region 13 of the pinhole lens member 10 and connected to the wireless communication unit 22, the IO interface 23 and the storage unit 27.

At the time of operation setting, the one-chip operation element 202 comes to control input/output of the wireless communication unit 22 based on the control information Dc0 to Dc2 and to control input/output of the liquid crystal display element 24, the light sensing element 29a, the pressure sensing element 29b and the imaging element 30 with the assistance of the IO interface 23. The control information Dc0 is, for example, data for setting an operation mode, the control information Dc1 is data for transferring a first image P11 at the time of setting a first image synthesis mode, and the control information Dc2 is data for transferring a second image P21 at the time of setting a second image synthesis mode. These pieces of the control information Dc0 to Dc2 are set by the external device 90.

The storage unit 27 (memory) is connected to the control unit 25, and the storage unit 27 temporarily stores the control information Dc0 to Dc2, the sensing information Dd1, Dd2, the imaging information Dg, and the like. The control information Dc0 to Dc2, the light sensing information Dd1, the intraocular pressure sensing information Dd2, and the imaging information Dg are read out to the control unit 25. The control unit 25 executes, for example, six operation modes based on the operation setting function from the external device 90. The six operation modes are brightness correction mode, alignment assistance mode, first image synthesis mode, second image synthesis mode, intraocular pressure sensing mode, and imaging mode.

The brightness correction mode refers to as an operation of controlling the brightness image P2 around the pinhole image P1 formed on the central fovea 88. The alignment assistance mode refers to as an operation of assisting optical axis/visual axis alignment when the pinhole contact lens 100 is worn. The first image synthesis mode refers to as an operation of forming the first image P11 around the pinhole image P1 formed on the central fovea 88. The second image synthesis mode refers to as an operation of forming the second image P21 around the retina outside the first image P11 around the pinhole image P1 formed on the central fovea 88. The intraocular pressure sensing mode refers to as an operation of acquiring the intraocular pressure during wearing of the pinhole contact lens 100. The imaging mode refers to as an operation of imaging an object during wearing of the pinhole contact lens 100.

The power supply unit 26 constituting an example of the electronic member 20 is connected to the control unit 25 to drive the wireless communication unit 22, the IO interface 23, the liquid crystal display element 24, the control unit 25, the storage unit 27, the light sensing element 29a and the pressure sensing element 29b. The power storage unit 28 (battery) is connected to the power supply unit 26, and is charged according to any one of a radio wave receiving (wireless induction feeding) system, an optical modulation wave feeding system and a solar power feeding system. The power storage unit 28 is composed of a large-capacity electrolytic capacitor. Of course, it may be composed of a rechargeable secondary battery.

The radio wave receiving system refers to as a system of transmitting power using electromagnetic induction among non-contact power transmission systems including non-contact charge. In the radio wave receiving system, a current is converted to an electromagnetic wave, on the power transmission side, which is transmitted to the power reception side. On the power reception side, the electromagnetic wave is received from the antenna element 21 and converted to a direct current, through a rectifier circuit, which is utilized as electric power. According to the frequency band conforming to the IEEE C95 standard, 3 kHz to 300 GHz can be utilized, which is regarded as a safe level even when the human body is exposed to electromagnetic waves.

As specific examples of the intended use of the radio wave receiving system, RFID (passive RFID tag) including FeliCa (registered trademark) is exemplified. The transmission distance is as long as several meters, but the transmission efficiency is poor. The optical modulation wave feeding system refers to as a power feeding system through non-contact power transmission using an optical modulation wave. The solar power feeding system refers to as a power feeding system through non-contact photoelectric conversion transmission using sunlight. In the case of the solar power feeding system, power can be charged at a bright place and can be stored. The load power of the electronic member 20 can be covered by the stored power at a dark place.

The external device 90 described above constitutes the smart contact system 900 shown in FIG. 6, includes antenna elements 91a and 91b, wireless communication units 92 and 93, a wireless power supply unit 94, a control unit 95, a power supply unit 96, a storage unit 97, an operation/display control unit 98 and a USB control unit 99, and can perform information processing generally. As for the functions of the components having the same names as those shown in FIG. 5, see the descriptions about FIG. 5. The wireless communication unit 92 performs data communication via the antenna element 91a and the antenna element 21 of the pinhole contact lens 100. The wireless communication unit 93 performs data communication via the antenna element 91b and the antenna element 911 of the smartphone 901. The power supply unit 96 is connectable to an external battery. An external storage device (such as an SD card) is connectable to the storage unit 97.

The operation/display control unit 98 is used for operations of input/output and display of these functions. In addition, various kinds of operation modes, as will be described later, are executed based on the operation settings input from the operation/display control unit 98. The wireless power supply unit 94 has an electromagnetic induction coil 94a and supplies wireless power to the pinhole contact lens 100. The control unit 95 controls input/output of the control information Dc0 to Dc2, the display information Dp, the imaging information Dg, the light sensing information Dd1 and the intraocular pressure sensing information Dd2. The USB control unit 99 is connected to an external device of the USB communication standard to perform data transfer control. They constitute the external device 90 of the smart contact system 900.

In the smart contact system 900, an IC chip of about 2.5 mm square of the Bluetooth standard is mounted on the pinhole lens member 10 (smart contact lens), thereby enabling direct short-range wireless communication of the same standard between the pinhole contact lens 100 and the smartphone 901, and also enabling data communication without the intermediary of the external device 90.

The lens member 11 having the diameter D4, which constitutes the pinhole lens member 10 capable of data communication with the external device 90 of the smart contact system 900, has a top surface (front surface) side lens member 111 and a rear surface (back surface) side lens member 112 constituting the lens member 11 as shown in FIG. 7, and has a sandwich structure in which the electronic member 20 is sandwiched by the top surface side lens member 111 and the rear surface side lens member 112. The lens member 111 and the lens member 112 are each made of a material that does not break even when bent. The lens member 11 preferably has high oxygen permeability.

The lens member 111 is molded into a hemispherical shape by an upper mold (cavity) having such a shape that a bowl is turned over, and the lens member 112 is molded into a dome shape (circular shape in plane view) by a lower mold (core) having a curved surface (curvature) along the surface shape of the cornea 80. In this embodiment, the one-chip operation element 202, the liquid crystal display element 24, the light sensing element 29a, the pressure sensing element 29b, the imaging element 30, and the like are mounted in advance on the light-shielding member 13'. The pinhole region 12 is also defined in a central part of the light shielding member 13'. Of course, the present invention is not limited to this, and the chip sealing method may be changed. The method of sticking the electronic member 20 onto the top surface or rear surface of the lens member 11 can be adopted.

In this embodiment, the front surface side lens member 111 and the back surface side lens member 112 are used to hold (cover) each of the front and back surfaces of the transparent region 14 in which the antenna element 21 including the first printed wiring part 201 is mounted, and each of the front and back surfaces of the light-shielding region 13 in which the one-chip operation element 202, the liquid crystal display element 24 including a second wiring region, the light sensing element 29a, the pressure sensing element 29b, the imaging element 30 and the like are mounted. Of course, the one-chip operation element 202, the liquid crystal display element 24 including the second wiring region, the light sensing element 29a, the pressure sensing element 29b, the imaging element 30, etc. are not limited to be provided in the light-shielding region 13, and they may be provided in the transparent region 14. In addition, a window may be provided in the lens member 112 or the like to deal with a detection element that requires direct contact with the cornea. Only a detection piece or the like of the detection element can be exposed from the window.

Thus, the light-shielding region 13, the transparent region 14, the antenna element 21, the one-chip operation element 202, the liquid crystal display element 24, the light sensing element 29a, the pressure sensing element 29b, the imaging element 30, etc. are integrally formed. As a result, the antenna element 21, the one-chip operation element 202, the liquid crystal display element 24, the light sensing element 29a, the pressure sensing element 29b, the imaging element 30, and the like can be completely/closely sealed by the lens members 111 and 112 and the like.

Moreover, when the pinhole contact lens 100 according to the present invention is put onto the cornea, light passes through the transparent region 14 while the light is shielded by the light-shielding region 13. Light at a center of the light limited by the light-shielding region 13 is converged to and passes through the pinhole region 12. At this time, the liquid crystal switching control by the liquid crystal display element 24 enables not only dynamic correction of the brightness around the pinhole image formed on the retina 86 shown in FIG. 4A but also reduction in gray image (ring image) caused by light diffraction.

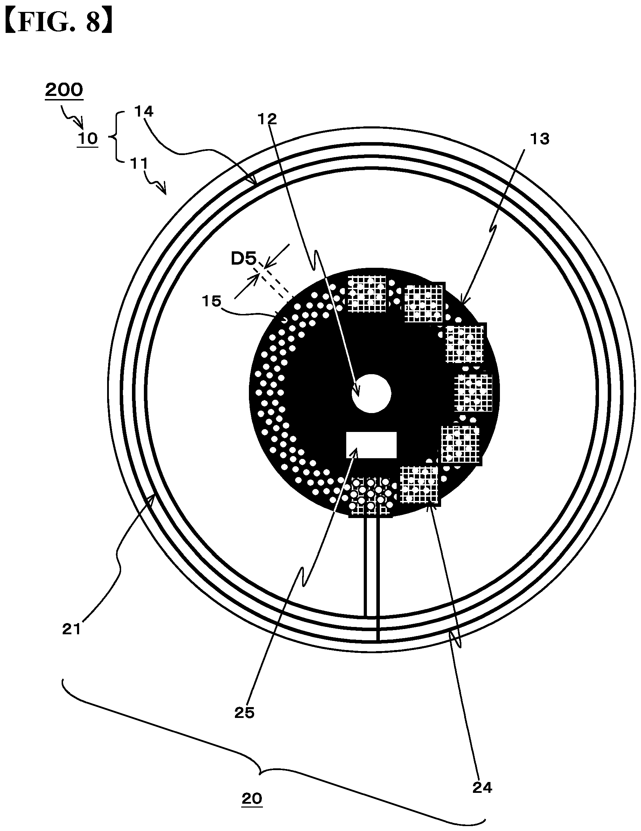

Next, the functions of the control unit 25 that executes various operation modes according to the second to seventh embodiments will be described with reference to FIG. 8 to FIG. 20.

<Second Embodiment>

In this embodiment, the control unit 25 executes the brightness correction mode. For this reason, the light-shielding region 13 of the pinhole contact lens 200 shown in FIG. 8 is devised. The light-shielding region 13 has the pinhole region 12 and a plurality of (many) holes 15. The liquid crystal display elements 24 are installed to close all or some of the holes 15. Each of the liquid crystal display elements 24 may be arranged either on the upper surface side or on the lower surface side of the light shielding member 13' as long as the holes 15 can be selected. Although, in FIG. 8, the liquid crystal display elements 24 are arranged in the right half for the sake of convenience in order to explain the distribution of the holes 15, but, of course, are arranged also in the left half. In the relationship between the holes 15 and the liquid crystal display elements 24, a plurality of holes 15 may be selected for one pixel, or one hole 15 may be selected for a plurality of pixels. The holes 15 can be selected with high accuracy.

As shown in FIG. 8, a plurality of holes 15 are randomly or regularly pierced between the hole peripheral edge of the pinhole region 12 and the outer peripheral edge of the light-shielding region 13, and has a function of allowing light from the external to be incident on the cornea 80 to secure brightness (night vision). In this embodiment, the plurality of holes 15 is distributed at the outer peripheral edge part of the light-shielding region 13, and no hole 15 is arranged at the outer peripheral edge part of the pinhole region. This is a configuration to obtain the maximum contrast of the pinhole image.

In particular, when the pinhole contact lens 100 is used at night or at a dark place, this is effective to secure the light amount. As the pitch (spacing) of the holes 15 is narrower, sufficient light can be secured. On the other hand, as the pitch of the holes 15 is wider, the contrast of the image can be improved. The number of the holes 15 is slightly increased as compared with that of the conventional fixed hole number type pinhole contact lenses. This is to widen the incident light amount range.

Moreover, in order to prevent diffraction of incident light, the shape of each of the holes 15 is preferably a perfect circle. The depth direction (penetration direction) of the holes 15 is inclined (along the dotted lines) toward the central fovea 88 (see FIGS. 4A and 4B) of the retina 86 as shown in FIGS. 4A and 4B and formed to be able to effectively concentrate incident light at the central fovea 88. The holes 15 are intended for securing the light amount in the central fovea 88. Further, this is due to the fact that visual signals of R, G and B colors are generated in the central fovea 88, and that the central fovea 88 is composed of cone photoreceptors and requires some degree of brightness for generation of the visual signals.

The diameter D5 of each of the holes 15 is selected to be smaller than the diameter of the pinhole region 12, and is selected, for example, in the range of 0.17 mm to 0.18 mm. This is because, when the diameter of the hole 15 is less than 0.17 mm, the hole diameter is too small to sufficiently take in incident light, so that night vision (brightness) cannot be secured. Also, this is because, when the diameter of the hole 15 is more than 0.18 mm, the diameter of the hole is too large, so that halo and glare are caused by light diffraction. In this embodiment, the holes 15 are pierced to increase by 10 to 20% more than those of the conventional fixed hole number type contact lenses.

In the relationship between the diameter D5 of the holes 15 and one pixel of the liquid crystal display element 24, when a size of the one pixel of the liquid crystal display element 24 is smaller than the diameter D5 of the holes 15, the diameter D5 of the holes 15 may exceed 0.18 mm. The number of pixels per unit area increases, and the pixel density increases, so that the formation density of the holes 15 can be covered.

Here, the optimum diameter D5 of the holes 15 can be determined from the relationship between the focal distance from the cornea 80 to the central fovea 88 of the retina 86 and the wavelength of light shown in FIG. 4A. Assuming that the diameter of the holes 15 is φ, that the wavelength of incident light is λ, and that the distance (eye axial length) from the cornea 80 to the central fovea 88 of the retina 86 is focal distance f, the diameter φ of the holes 15 is determined based on the following equation (1).

[Mathematical Equation 1]

$$\phi = \sqrt{2\lambda f}$$
$$= 0.03679\sqrt{f} \quad \cdots\cdots(1)$$

Here, since the focal distance f is generally 23 mm to 24 mm, and, for example, if the numerical value 23 mm is substituted into the above equation (1), 0.176 mm can be obtained as the optimum diameter φ of the holes 15. Further, when the numerical value 24 mm is substituted as the focal distance f into the above equation (1), 0.180 mm can be obtained as the optimum diameter φ of the holes 15.

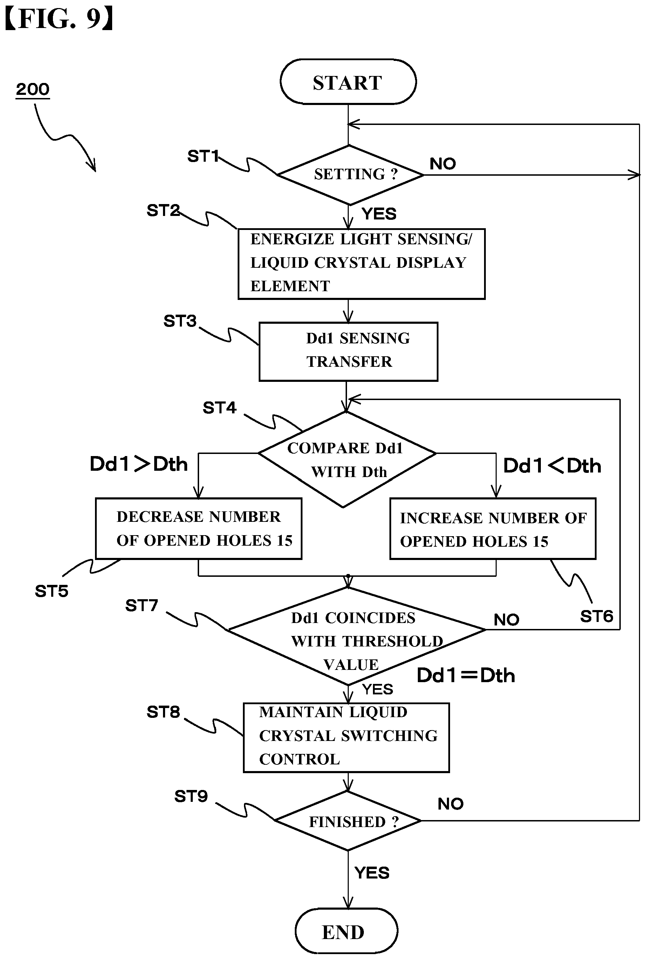

Here, with reference to FIG. 9, a control example of the brightness correction mode in the control unit 25 will be described. In this embodiment, as shown in FIG. 8, the liquid crystal display elements 24 each having a matrix shape are provided in the whole or a part of the light-shielding region 13 of the pinhole lens member 10. In this embodiment, the liquid crystal display elements 24 are provided on the incident light side of the light-shielding region 13. Furthermore, a plurality of fine holes 15 is provided on the outer peripheral edge of the light-shielding region 13 of the pinhole lens member 10, and the control unit 25 performs liquid crystal switching control on the liquid crystal display elements 24 arranged on the light-shielding region 13 to control the liquid crystal display elements 24 so as to select the hole(s) 15. The power supply will be described in the case of the radio wave receiving system. In this embodiment, in conformity with the IEEE C95 standard, when the effective radiated power (EIRP) is 40 dBm, the separation distance is 11 cm, and the frequency is 1.8 GHz, the received power is about 6 mW/cm.

In this embodiment, it is assumed that the large-capacity power storage unit 28 is provided. First, in a step ST1 shown in FIG. 9, the control unit 25 waits for the setting of the brightness correction mode. When the external device 90 approaches the pinhole contact lens, the wireless communication unit 22 and the power supply unit 26 operate. When the power supply unit 26 generates a voltage V, the power storage unit 28 is charged with the voltage V. When the power is supplied from the external device 90 and the brightness correction mode is set, in a step ST2, the control unit 25 energizes the liquid crystal display element 24 and the light sensing element 29a. By this energization, the light sensing element 29a senses the light transmitted through the pinhole region 12 and outputs the light sensing information Dd1 to the control unit 25.

Thereafter, in a step ST3, the control unit 25 receives the light sensing information Dd1 transferred from the light sensing element 29a. The control unit 25 controls the brightness around the pinhole image based on at least either one of the light sensing information Dd1 and the control information Dc0 from the wireless communication unit 22.

For example, in a step ST4, the control unit 25 compares a light amount Dm based on the light sensing information Dd1 with a threshold value Dth based on the control information Dc0. The threshold value Dth is included in the control information Dc0, is set by the external device 90, and varies depending on the operation mode, sensing conditions, and the like. When the light amount Dm is larger than the threshold value Dth (Dm > Dth), the process shifts to a step ST5 in which the liquid crystal display elements 24 are controlled (liquid crystal switching control) to select the holes 15 to decrease the number of the opened holes 15.

In a step ST7, the control unit 25 determines whether or not the light amount Dm and the threshold value Dth coincide with each other. If they do not coincide with each other, the process returns to the step ST4 in which the control unit 25 compares the light amount Dm based on the light sensing information Dd1 with the threshold value Dth based on the control information Dc0 again. When the light amount Dm is smaller than the threshold value Dth (Dm < Dth), the process shifts to a step ST6 in which the liquid crystal switching control is performed so as to deselect the holes 15 to increase the number of the opened holes 15.

When the light amount Dm and the threshold value Dth coincide with each other (Dm = Dth) in the step ST7, the control unit 25 maintains the liquid crystal switching control in a step ST8. Thereafter, the control unit 25 senses whether or not the process is finished, in a step ST9. When the power is turned off, the control is finished. When the power is turned on, the process returns to the step ST1 to continue the control.

According to the second embodiment, it becomes possible to selectively control a plurality of fine holes 15 pierced on the outer peripheral edge of the light-shielding region 13. So, the brightness around the pinhole image can be precisely controlled in response to the bright environment and the dark environment. This enables the night vision (brightness) around the pinhole image to be adjusted.

<Third Embodiment>

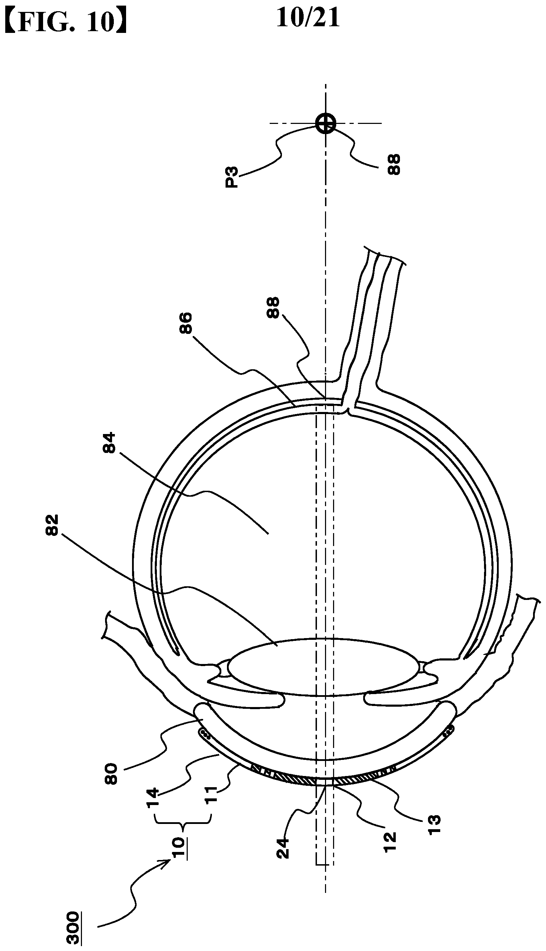

In the third embodiment, the control unit 25 executes the alignment assistance mode. Therefore, the liquid crystal display element 24 is formed in the pinhole region 12 as in the pinhole contact lens 300 shown in FIGS. 10A and 10B. For example, the opening part itself for forming the pinhole image on the retina 86 of the wearer is formed from the liquid crystal display element 24. For the liquid crystal display element 24, for example, PLCD liquid crystal can be used. The PLCD liquid crystal controls transmission of light based on the presence or absence of charge, and, in the case of the reverse type, transmits light in the absence of charge. Of course, the liquid crystal display element 24 is not limited to the PLCD liquid crystal.

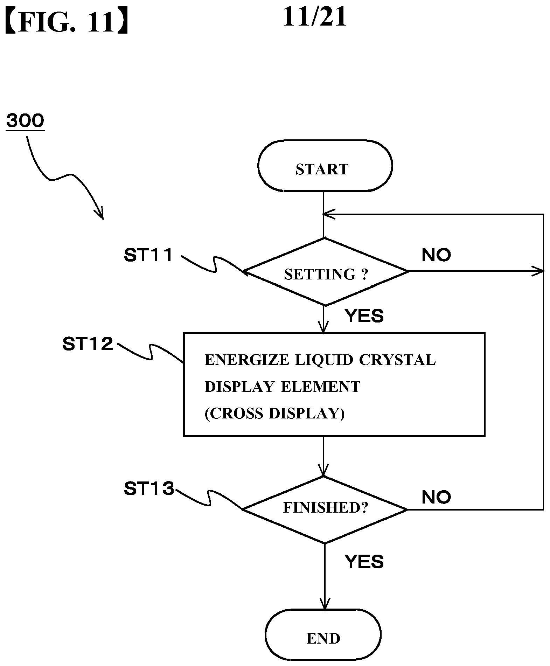

In this embodiment, first, in a step ST11 shown in FIG. 11, the control unit 25 waits for the setting of the alignment assistance mode. When the external device 90 approaches the pinhole contact lens, the wireless communication unit 22 and the power supply unit 26 operate. When the power supply unit 26 generates a voltage V, the power storage unit 28 is charged with the voltage V.

When the power is supplied from the external device 90 and the alignment assistance mode is set, the control unit 25 energizes the liquid crystal display element 24 in a step ST12. The liquid crystal display element 24 displays an alignment mark in the pinhole region 12. The alignment mark is displayed in a cross shape. Hereinafter, the cross-shaped alignment mark is referred to as a cross mark P3. The wearer operates and aligns the pinhole contact lens so that the visual axis (eye axis/optical axis) coincides with the cross mark P3. Of course, not only the cross mark P3 but also textual information and characters may be displayed. For example, a character printed with a character pattern (picture and/or text) is displayed on/off. In addition, the diameter D1 of the pinhole region may be adjusted to control the intensity of the light flux (beam light) provided by the pinhole.

Thereafter, it is sensed in a step ST13 whether or not the process is finished. When the power is turned off, the control is finished. When the power is turned on, the process returns to step ST11 to continue the control. As a result, the pinhole contact lens 100 can be put, with good reproducibility, onto the eye axis (visual axis/optical axis) of the wearer. In addition, if an open state is established when the liquid crystal power is turned off, the current pinhole effect can be maintained even in the case of failure of all the liquid crystal functions.

In addition, the opening part of the pinhole region 12 may be composed of a matrix-shaped high-density liquid crystal display element, and control may be performed so as to appropriately select the number of pixels and arrangement positions thereof to change the diameter D1 of the pinhole region 12 in the range of 1.0 mm to 1.6 mm. Thus, it is possible to electronically control the addition power in the range of approximately 1.00 to 3.00D depending on the change in size of the diameter D1.

<Fourth Embodiment>

In this embodiment, the control unit 25 executes the first image synthesis mode. Therefore, the display unit of the pinhole contact lens 400 shown in FIG. 5 is composed of a surface (self) light-emitting element 241 instead of the liquid crystal display element 24 and displays an image based on the control information Dc0 to Dc2 received by the wireless communication unit 22, and the image is formed around the pinhole image. The surface-emitting element 241 displays virtual images by various applications based on the display information Dp added to the control information Dc1.

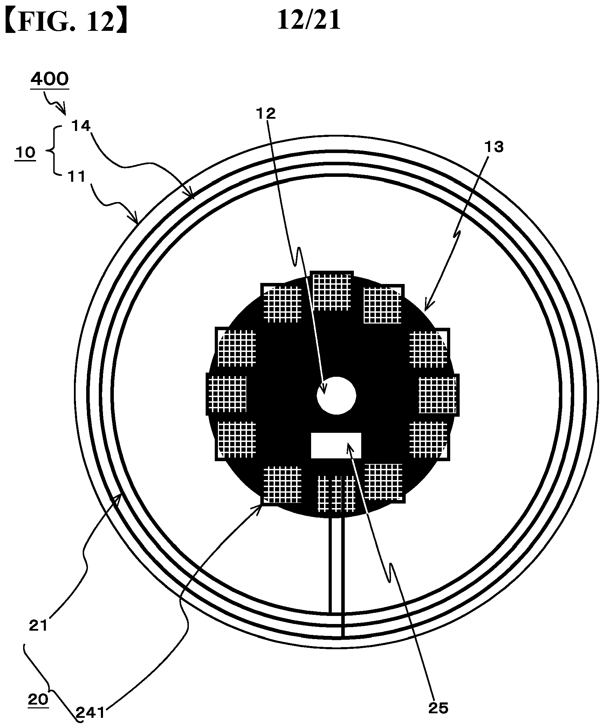

In this embodiment, for example, twelve surface-emitting elements 241 shown in FIG. 12, which constitute the display unit, are provided on the cornea-wearing side of the light-shielding region 13 of the pinhole lens member 10. The surface-emitting elements 241 are provided radially at predetermined intervals around the light-shielding region 13. For example, they are provided radially at intervals of 30°. As a result, it is possible to uniformly and equally synthesize and display a matrix image in an annular shape (circular shape). Each of the surface-emitting elements 241 has a red light emitting element, a blue light emitting element, and a green light emitting element which constitute examples thereof, and has a matrix shape of M rows $\times$ N columns. M and N shown in FIG. 12 are each 5 (M = 5, N = 5). Of course, M and N are not limited to this. M may be 1, 2, 3 or the like, and N may be 1, 2, 3 or the like.

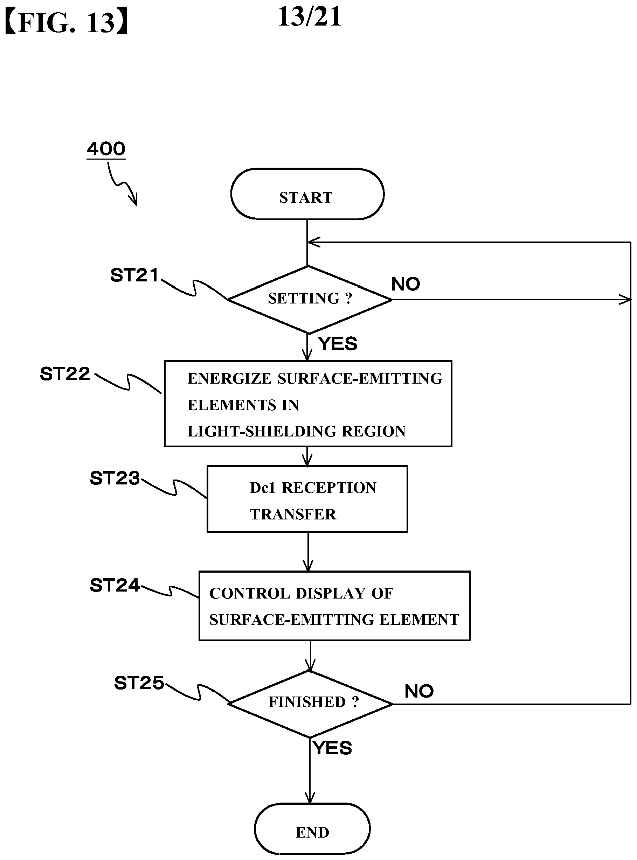

In this embodiment, first, in a step ST21 shown in FIG. 13, the control unit 25 waits for the setting of the first image synthesis mode. When the external device 90 approaches the pinhole contact lens, the wireless communication unit 22 and the power supply unit 26 operate. When the power supply unit 26 generates a voltage V, the power storage unit 28 is charged with the voltage V.

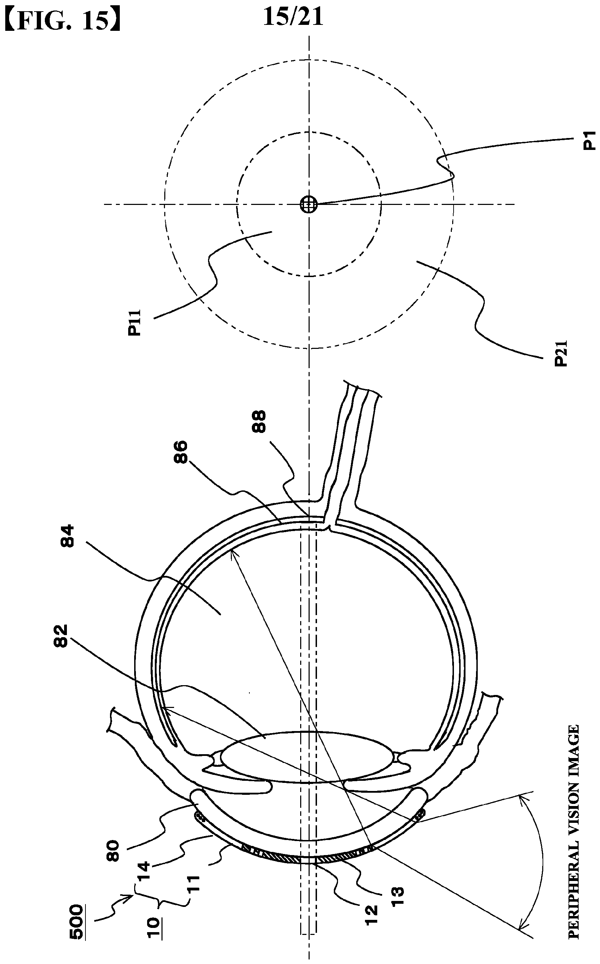

When the power is supplied from the external device 90 and the first image synthesis mode is set, the control unit 25 energizes the twelve surface-emitting elements 241 in the light-shielding region 13 in a step ST22. Thereafter, in a step ST23, the control unit 25 inputs the control information Dc1 from the wireless communication unit 22. In a step ST24, the control unit 25 executes control to display the first image P11 around the pinhole image based on the control information Dc1. The first image P11 is color textual information, character pattern, or moving image. For example, the first image P11 is displayed annularly along the outer peripheral edge of the pinhole image P1 shown in FIG. 15.

Thereafter, it is sensed in a step ST25 whether or not the process is finished. When the power is turned off, the control is finished. When the power is turned on, the process returns to the step ST21 to continue the control. By the display control of the surface-emitting elements 241, it is possible to synthesize a color virtual image (image) with respect to a natural-color pinhole real image to form the images on the retina.

As described above, according to the fourth embodiment, it is possible to form a real image by the pinhole region 12 on the retina 86 while, with respect to the real image, synthesizing a virtual image (first image P11) based on the control information Dc1 on the retina 86. The first image P11 can be displayed annularly around the real image.

As a result, it is possible to make the brain of the contact lens wearer recognize the first image P11 based on the control information Dc1 in real time while maintaining the pinhole effect. Therefore, it is possible to provide a smart contact lens which enables quality of a pinhole image to be improved, a correction of all of myopia or hyperopia, astigmatism and presbyopia to cope by utilizing the original depth of focus provided by the pinhole region 12 and high functionality to attain.

<Fifth Embodiment>

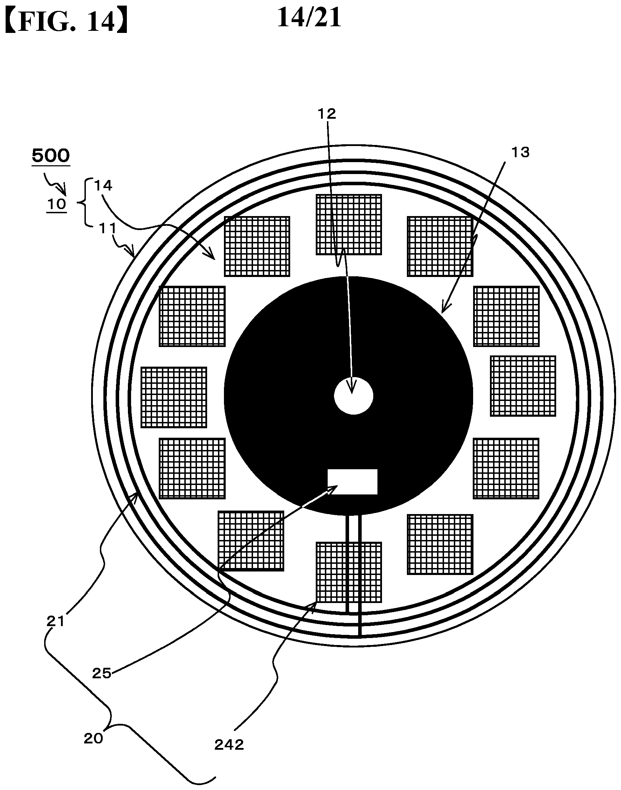

In this embodiment, the control unit 25 executes the second image synthesis mode. Therefore, as in the case of the pinhole contact lens 500 shown in FIG. 14, liquid crystal display elements 24 each having a matrix-shape, which constitute an example of the electronic member 20, are provided in the whole or a part of the transparent region 14 of the pinhole lens member 10. For example, twelve sets of $10 \times 10$ pixel liquid crystal display elements 242 constituting the liquid crystal display elements 24 are provided between the antenna element 21 and the light-shielding region 13 radially at intervals of 30° around the transparent region 14. Each of the liquid crystal display elements 242 has a dot color film member of three colors of red, blue and green.

In the sectional view of an eyeball shown in FIG. 15, the peripheral vision images by the pinhole region 12 and the light-shielding region 13 are transmitted through the transparent region 14 provided around the light-shielding region 13 and made incident on the cornea 80, pass through the pupil, enter a vitreous body 84 within the eyeball, and are formed on the retina 86 on the rear surface side of the vitreous body 84. Therefore, the peripheral vision image by the transparent region 14 and the color virtual image (second image P21) can be synthesized in the peripheral vision of the natural-color pinhole real image obtained by the pinhole region 12 to form the images on the retina 86.

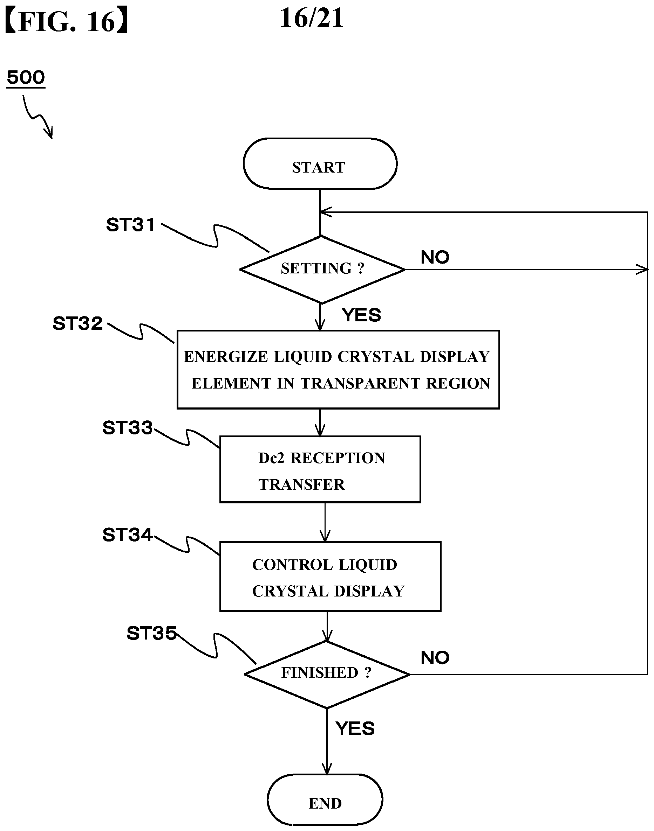

In this embodiment, first, in a step ST31 shown in FIG. 16, the control unit 25 waits for the setting of the second image synthesis mode. When the external device 90 approaches the pinhole contact lens, the wireless communication unit 22 and the power supply unit 26 operate. When the power supply unit 26 generates a voltage V, the power storage unit 28 is charged with the voltage V.

When the power is supplied from the external device 90 and the second image synthesis mode is set, the control unit 25 energizes each of the liquid crystal display elements 242 in the transparent region 14 in a step ST32. Thereafter, in a step ST33, the control unit 25 inputs the control information Dc2 from the wireless communication unit 22. In a step ST34, the control unit 25 executes liquid crystal display control for displaying the second image P21 (virtual image) around the pinhole image based on the control information Dc2. The second image P21 constitutes textual information and a character. For example, the liquid crystal display elements 242 display an animated character pattern (picture and/or text) or a still image thereof.

The second image P21 is, for example, displayed annularly along the outer peripheral edge of the first image P11 displayed annularly along the outer peripheral edge of the pinhole image P1 shown in FIG. 15. Because the retina 86 around the central fovea 88 is composed of rod photoreceptors, a clear image may not be visually recognized. However, when the first and second image synthesis modes are superimposed, it becomes possible to synthesize and display two images in such a form that the second image P21 in the second image synthesis mode complements the brightness of the first image P11 in the first image synthesis mode.

Thereafter, it is sensed in a step ST35 whether or not the process is finished. When the power is turned off, the control is finished. When the power is turned on, the process returns to step ST31 to continue the control.

As described above, according to the fifth embodiment, it becomes possible to synthesize the peripheral vision image by the transparent region 14 and the color virtual image (second image P21) in the peripheral vision of the real image by the pinhole region 12 to form the images on the retina 86. The second image P21 can be displayed annularly around the real image.

As a result, it is possible to make the brain of the contact lens wearer recognize the second image P21 based on the control information Dc2 in real time while maintaining the pinhole effect. Therefore, it is possible to provide a smart contact lens which enables quality of a pinhole image to be improved, a correction of all of myopia or hyperopia, astigmatism and presbyopia to cope by utilizing the original depth of focus provided by the pinhole region 12 and high functionality to attain.

Moreover, all of the pinhole region 12, light-shielding region 13 and transparent region 14 of the pinhole lens member 10 can be formed of the liquid crystal display elements 24. Thus, the pinhole contact lens itself can be constructed by the film-like lens member 11 and the liquid crystal display elements 242. A so-called transparent display in which the other side (object side) can be seen can be configured.

<Sixth Embodiment>

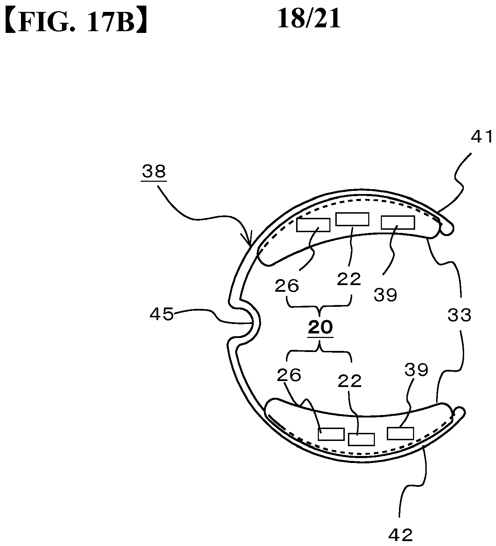

In this embodiment, as shown in FIG. 17B, the electronic member 20 and a drug containing unit 33 are mounted on a C-shaped base main body 38 which can be worn in the eyelid of a person. The electronic member 20 is at least a part of the wireless communication unit 22, the liquid crystal display element 24, the sensing unit (such as the light sensing element 29a and the pressure sensing element 29b), the control unit 25 and the power supply unit 26 as shown in FIG. 5. For example, the power supply unit 26 and the wireless communication unit 22 are mounted on the base main body 38.

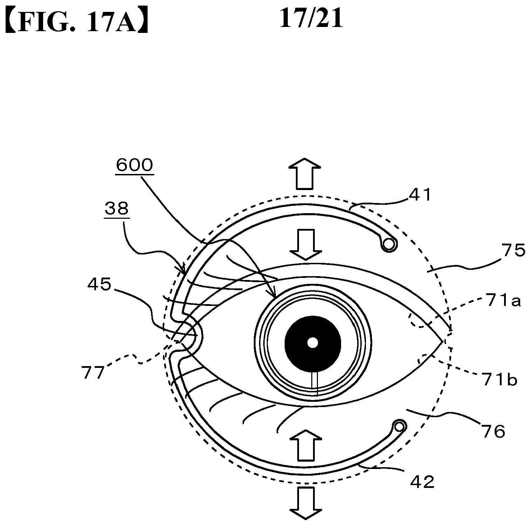

In the plan view of the pinhole contact lens 600 shown in FIG. 17A, the electronic member 20 and the drug containing unit 33 are omitted for the sake of explanation. The base main body 38 has an arc shape that can be abutted between the upper end of the surface outer peripheral edge part of the wearer's cornea and the base part of an upper eyelid capsule 71a on the rear side of an upper eyelid 75. An arcuate part 42 has an arc shape that can be abutted between the lower end of the surface outer peripheral edge part of the cornea and the base part of a lower eyelid capsule 71b on the rear side of a lower eyelid 76. The base main body 38 has a loop shape with one end opened, and has a lower chord-shaped first arcuate part 41, an upper chord-shaped second arcuate part 42 and a substantially U-shaped connection part 45. The free end of each of the arcuate parts 41 and 42 is rounded so as not to damage the inside of the eye.

The drug containing units 33 shown in FIG. 17B are those which can be detachably attached to the base main body 38 (Drug Delivery System: DDS/ophthalmologic drug delivery system) and automatically supply the drug solution to sites including the eyeball and the conjunctiva for a long time. In this embodiment, the automatic supply of the drug is performed by piezoelectric elements 39 as shown. The piezoelectric elements 39 are automatically controlled, for example, by the control unit 25 shown in FIG. 5.

The connection part 45 constitutes a portion connecting the arcuate part 41 and the arcuate part 42, and has a size enough to surround a zonule 77 located on the ear side of the eyelid shown in FIG. 17A. The zonule 77 is present at an ear-side boundary between the upper eyelid capsule 71a and the lower eyelid capsule 71b in the conjunctiva of a human. The connection part 45 is recessed inward from a virtual outer peripheral line when the arcuate parts 41 and 42 are continuous. The connection part 45 is configured to apply a mutual repulsive force upward to the arcuate part 41 and downward to the arcuate part 42, as indicated by the outlined arrows in the figure.

The base main body 38 is, for example, a metal such as gold (Au) or stainless steel, a resin, a glass fiber, or the like. The thickness thereof is about 0.1 mm to 2.0 mm. The base main body 38 is preferably as thin as possible so long as the mutual repulsive force against the arcuate parts 41 and 42 can be secured to some extent, in order to prevent the feeling of foreign subject insertion.

The base main body 38 is stabilized in the capsules while a light tension for opening it outward is applied to a turnback site at the central part thereof as shown by the outlined arrows in FIG. 17A. Further, the base main body 38 not only avoids the zonule, but also plays a role of preventing the main body from rotating in the capsules, so that the electronic member 20 can be maintained at a stable position.

When a system of putting a part of the electronic member 20 on the base main body 38 is adopted, the electronic member 20 is stably stored deep inside the eyelid capsules, and a short-range feeding system by radio waves can be adopted, for example, between the base main body 38 and the pinhole lens member 10.

As a result, the power supply unit 26 having a sufficient capacity can be installed in the base main body 38, so that it is possible to construct an independent type smart contact lens which performs information processing while giving and receiving power within the eye as compared with the backscatter system.

In this embodiment, in the pinhole contact lens 600 shown in FIG. 17A, the control unit 25 shown in FIG.

5 executes the intraocular pressure sensing mode. The control unit 25 comes to control writing of the intraocular pressure sensing information Dd2 in the storage unit 27 at constant time intervals, or to automatically control the amount of eyedrops or the like to be released slowly from the piezoelectric element 39 (another passive element) based on the intraocular pressure sensing information Dd2 and the control information Dc0.

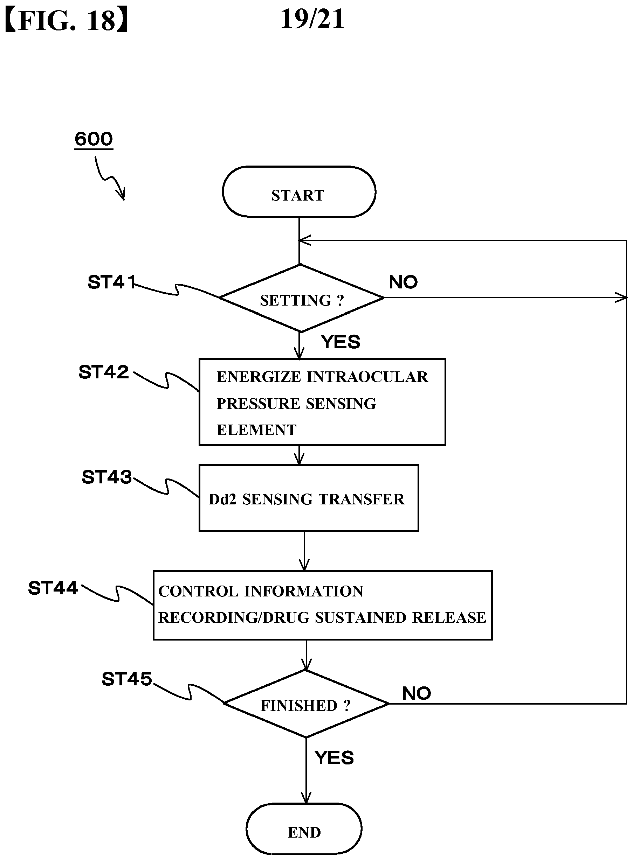

In this embodiment, first, in a step ST41 shown in FIG. 18, the control unit 25 waits for the setting of the intraocular pressure sensing mode. When the external device 90 approaches the pinhole contact lens of the wearer, the wireless communication unit 22 and the power supply unit 26 operate. When the power supply unit 26 generates a voltage V, the power storage unit 28 is charged with the voltage V. At this time, power may be supplied to the pinhole lens member 10 from the power supply unit 26 mounted on the base main body 38 shown in FIG. 17B.

When power is supplied from the external device 90 and the intraocular pressure sensing mode is set, the control unit 25 energizes the pressure sensing element 29b in the light-shielding region 13 in a step ST42. In response to the energization, the pressure sensing element 29b outputs the intraocular pressure sensing information Dd2 to the control unit 25. The pressure sensing element 29b is not limited to be provided in the light-shielding region 13, and it may be provided in the base main body 38 shown in FIG. 17B.

Thereafter, in a step ST43, the control unit 25 inputs the control information Dc2 and the intraocular pressure sensing information Dd2 from the wireless communication unit 22. In a step ST44, the control unit 25 controls, for example, the piezoelectric element 39 or the like shown in FIG. 17B based on the control information Dc2 and the intraocular pressure sensing information Dd2. In this control, the intraocular pressure sensing information Dd2 is stored in the storage unit 27 at constant time intervals, or drug sustained release control is executed. In the drug sustained release control, a target value thereof is set, and the piezoelectric element 39 is controlled to be driven so as to attain sustained release of an eye drop in an optimal sustained release amount corresponding to the intraocular pressure from the drug containing unit 33.

Thereafter, it is sensed in a step ST45 whether or not the process is finished. When the power is turned off, the control is finished. When the power is turned on, the process returns to the step ST41 to continue the control. This enables a specific eye disease, glaucoma, and the like to be treated while forming a pinhole image on the retina 86.

In the treatment of glaucoma, drainage control of the aqueous humor under the cornea can be performed. The sensing unit is not limited to the pressure sensing element 29b, and may be an element that senses the value of sugar contained in tears (blood glucose level). For example, an enzyme (glucose oxidase) electrode film can be used in the sensing unit. The target to be sensed may be a heart rate. A drug solution which lowers the blood glucose level can be released slowly from the drug containing unit 33 or the like according to the sensed information on the blood glucose level. Moreover, the smart contact lens can be used in an application (remote diagnosis treatment comprehensive system) in which the sensing information on the blood glucose level is transferred to a medical institution via the smartphone 901. This makes it possible to non-invasively remotely monitor the blood glucose level of a patient with diabetes and to control the drainage of aqueous humor while remotely monitoring the intraocular pressure of a patient with glaucoma. When configuring the smart contact system for these applications, the external device 90 may be composed of a smart contact dedicated control device so that information obtained from this dedicated control device may be transferred to the medical institution via the smartphone 901. The smart contact system may also be configured so that prescription (treatment) instruction data may be transferred from the medical institution to the smartphone 901 of the pinhole contact lens wearer.

<Seventh Embodiment>

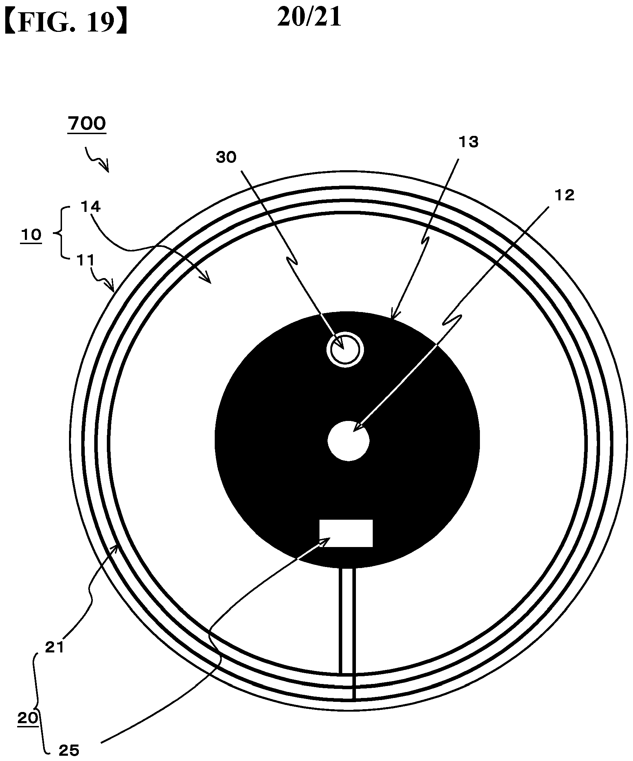

In this embodiment, in the pinhole contact lens 700 shown in FIG. 19, the imaging element 30 (camera) is provided on the pinhole lens member 10. For example, the imaging element 30 is arranged on the top surface side of the light-shielding region 13, and the shutter mechanism can adopt a system of sensing blinks by the upper eyelid and the lower eyelid. As the shutter mechanism, the detection technique of the eyelid closing described in US Patent Application Publication No. 2016/0097940 can be applied.

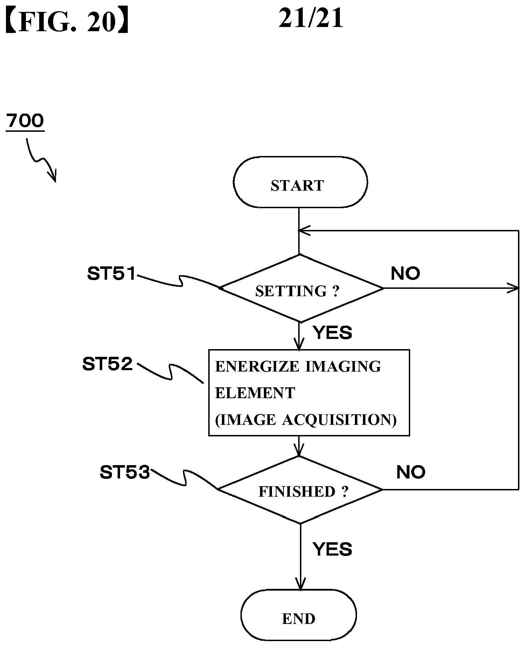

In FIG. 20, the control unit 25 shown in FIG. 5 executes the imaging mode. First, in a step ST51, the control unit 25 waits for the setting of the imaging mode. When the external device 90 approaches the pinhole contact lens of the wearer, the wireless communication unit 22 and the power supply unit 26 operate. When the power supply unit 26 generates a voltage V, the power storage unit 28 is charged with the voltage V.

When the power is supplied from the external device 90 and the imaging mode is set, the control unit 25 energizes the imaging element 30 in the light-shielding region 13 in a step ST52. In response to this energization, the imaging element 30 is put into operation. In this embodiment, blinks are sensed by a sensing means (not shown) to capture a real image of the object, and the imaging information Dg thereof is written in the storage unit 27 via the control unit 25. The imaging information Dg is read out to the outside through the wireless communication unit 22.

Thereafter, it is sensed in a step ST53 whether or not the process is finished. When the power is turned off, the control is finished. When the power is turned on, the process returns to the step ST51 to continue the control. This enables the imaging mode to be executed while forming a pinhole image on the retina, upon setting of the imaging mode.

In this embodiment, the case where the imaging element 30 is arranged on the top surface side of the light-shielding region 13 to image the object has been described, but the present invention is not limited thereto. The imaging element 30 may be arranged on the rear side of the light-shielding region 13 to image the fundus. It becomes possible to acquire an image of the peripheral retina including the central fovea 88. At this time, the surface-emitting element 241 can be used for white display to brighten an imaging region, and simple fundus examination information can be acquired. The present invention will be able to contribute to the assistance of ophthalmologic medical care.

Thus, while the present invention has been disclosed with respect to specific preferred embodiments, those skilled in the art should understand that the present invention extends beyond the specific disclosed embodiments to other alternatives and/or to the use, apparent variations and equivalents of the present invention. Additionally, while numerous variations of the present invention have been illustrated and described in detail, other variations that fall within the scope of the present invention can be readily conceived by those skilled in the art based on the present disclosure. For example, the light-shielding region 13 is configured to have a gradation. In this configuration, a light-shielding peripheral region having a light shielding rate higher (different) than that of the light-shielding region 13 is provided between the light-shielding region 13 and the transparent region 14. This is to ensure the brightness of the retina at the peripheral edge of the pinhole image.

Also, particular features of the embodiments and aspects thereof can be variously combined or partially combined, which are also considered to fall within the scope of the present invention. Accordingly, it should be understood that various features of the disclosed embodiments and aspects thereof are combined or replaced to form different modes of the disclosed invention. For example, in the fourth embodiment, the light transmitted through the pinhole region may be sensed by the sensing unit, and the display of the self-emitting element may be controlled based on the information from the sensing unit.

In addition, an external world image (real image) is taken by the imaging element 30 (camera), and is reproduced by the self-emitting elements for R, G and B colors based on the first image synthesis mode described in the second embodiment. Or, instead of the cross mark described in the third embodiment, an external world image is displayed by the liquid crystal display element 24, and is projected onto the retina 86. This makes it possible to emphasize the pinhole image, and additionally enables even weak-sighted persons whose cornea, crystalline lens and vitreous body hardly function to visually recognize the external world image. As a result, it is possible to configure a smart contact lens with simple elements as compared with a retina projection system of a QD laser beam scanning type of glasses type.

Thus, the technical scope of the present invention disclosed in the present application is not limited to the specific disclosed embodiments described above, but is determined only by fair interpretation of the Claims.

[EXPLANATION OF CODES]

10: Pinhole Lens Member
11: Lens Member
12: Pinhole Region
13: Light-shielding Region
14: Transparent Region
15: Hole
20: Electronic Member
21, 91a, 91b, 911: Antenna Element
22, 92, 93: Wireless Communication Unit
23: IO Interface
24: Liquid Crystal Display Element
25, 95: Control Unit
26, 96: Power Supply Unit
27, 97: Storage Unit
28: Power Storage Unit
29a: Light Sensing Element
29b: Pressure Sensing Element
30: Imaging Element
38: Base Main Body
90: External Device
94: Wireless Power Supply Unit
94a: Electromagnetic Induction Coil
98: Operation/Display Control Unit
99: USB Control Unit
1 (100 to 700): Pinhole Contact Lens
900: Smart Contact System
901: Smartphone

Claims

1. A pinhole contact lens comprising:

a pinhole lens member which contains a lens member fittable onto a cornea of a person, a pinhole region having a circular shape and arranged approximately in a center of the lens member, the pinhole region forming a pinhole image on a retina of a wearer, a light-shielding region provided on an outer peripheral side of the pinhole region and shielding light, and a transparent region provided on an outer peripheral side of the light-shielding region and transmitting the light; and
an electronic member which is arranged on any one of the pinhole region, the transparent region and the light-shielding region and electrically performs an optical processing on any one of the light transmitted via the transparent region and the light absorbed into the light-shielding region on the basis of any control information, with respect to light passing through the pinhole region, to be applied on the pinhole image.

2. The pinhole contact lens according to Claim 1, wherein the electronic member arranged on the pinhole lens member contains:

a wireless communication unit which has an antenna element to transmit and receive the control information;
a display unit which forms an image based on the control information around the pinhole image;
a sensing unit which senses the light transmitted through the pinhole region; and
a control unit which controls brightness around the pinhole image based on at least either one of sensing information from the sensing unit and the control information from the wireless communication unit.

3. The pinhole contact lens according to Claim 2, wherein the display unit contains a liquid crystal display element which is provided in a whole or a part of the pinhole region of the pinhole lens member and has a matrix shape.

4. The pinhole contact lens according to Claim 3, wherein the display unit forms an opening part for forming the pinhole image on the retina of the wearer in the pinhole region.

5. The pinhole contact lens according to Claim 4, wherein the display unit displays an alignment mark in the pinhole region.

6. The pinhole contact lens according to Claim 2, wherein the display unit contains a liquid crystal display element which is provided in a whole or a part of the light-shielding region of the pinhole lens member and has a matrix shape.

7. The pinhole contact lens according to Claim 6, wherein a plurality of fine holes is provided on an outer peripheral edge of the light-shielding region of the pinhole lens member, and
wherein the control unit of the pinhole lens member performs liquid crystal switching control on the display unit arranged in the light-shielding region to control the display unit so as to select the holes.

8. The pinhole contact lens according to Claim 2, wherein the display unit contains a liquid crystal display element which is provided in a whole or a part of the transparent region of the pinhole lens member and has a matrix shape.

9. The pinhole contact lens according to Claim 3 or 8, wherein the liquid crystal display element includes a dot color film member of three colors of red, blue and green.

10. The pinhole contact lens according to Claim 2, wherein the pinhole lens member includes a power supply unit which drives the wireless communication unit, the sensing unit, the display unit and the control unit, and
wherein the power supply unit includes a power storage element having any one of a wireless induction feeding system, an optical modulation wave feeding system, and a solar power feeding system.

11. The pinhole contact lens according to Claim 2, wherein the control unit contains a microprocess element which is provided in the light-shielding region of the pinhole lens member, and connected to the wireless communication unit, the display unit and the sensing unit to control input/output of the wireless communication unit, the sensing unit, and the display unit based on the control information.

12. The pinhole contact lens according to Claim 1, wherein the electronic member contains a self-emitting element arranged at least on a cornea-wearing side of the light-shielding region of the pinhole lens member, and performs display control on light from the self-emitting element based on control information to be applied on the pinhole image.

13. The pinhole contact lens according to Claim 12, wherein the electronic member contains:

a wireless communication unit which includes an antenna element to transmit and receive the control information;
a display unit which includes a red light emitting element, a blue light emitting element, and a green light emitting element, and forms an image based on the control information around the pinhole image; and
a control unit which controls the display unit to form an image based on the control information from the wireless communication unit around the pinhole image.

14. The pinhole contact lens according to Claim 2 or 13, wherein the wireless communication unit contains an antenna element which is provided along an outer peripheral edge of the transparent region of the pinhole lens member and has a loop shape.

15. The pinhole contact lens according to Claim 13, wherein the control unit contains a microprocess element which is provided in the light-shielding region of the pinhole lens member, and connected to the wireless communication unit and the display unit to control input/output of the wireless communication unit and the display unit based on the control information.

**16.** The pinhole contact lens according to Claim 13, wherein the pinhole lens member includes a power supply unit which drives the wireless communication unit, the display unit, and the control unit, and wherein the power supply unit includes a power storage element having any one of a wireless induction feeding system, an optical modulation wave feeding system and a solar power feeding system.

**17.** The pinhole contact lens according to Claim 16, wherein the electronic member in at least a part of the wireless communication unit, the display unit, the control unit, and the power supply unit is mounted on a C-shaped base main body which is wearable in an eyelid of a person.

**18.** The pinhole contact lens according to Claim 1 or 12, wherein the pinhole lens member is provided with an imaging element.

**19.** The pinhole contact lens according to Claim 1 or 12, wherein the pinhole lens member is provided with a storage element.

**20.** The pinhole contact lens according to Claim 1 or 12, wherein the pinhole lens member is provided with a pressure sensing element.

**21.** The pinhole contact lens according to Claim 1 or 12, wherein the lens member contains a top surface side lens member and a rear surface side lens member, and
wherein the electronic member has a sandwich structure in which the electronic member is sandwiched by the top surface side lens member and the rear surface side lens member.

**22.** The pinhole contact lens according to Claim 1 or 12, wherein the pinhole lens member is composed of at least either one of a soft contact lens and a hard contact lens.

**23.** A smart contact system comprising:

the pinhole contact lens according to any one of Claims 1 to 22;
an external device capable of data communication with the pinhole contact lens; and
a smartphone capable of data communication with the external device.

【FIG. 1】

【FIG. 2】

【FIG. 3】

【FIG. 4】

【FIG. 5】

【FIG. 6】

【FIG. 7】

【FIG. 8】

## 【FIG. 9】

<u>200</u>

```
                    ┌──────────┐
                    │  START   │
                    └────┬─────┘
                         │
         ST1        ╱────┴────╲        NO
          ┌───────<  SETTING ?  >──────────────────┐
                    ╲─────────╱                     │
                         │ YES                      │
         ST2   ┌─────────┴──────────┐               │
          ┌──→ │ ENERGIZE LIGHT     │               │
               │ SENSING/           │               │
               │ LIQUID CRYSTAL     │               │
               │ DISPLAY ELEMENT    │               │
               └─────────┬──────────┘               │
         ST3   ┌─────────┴──────────┐               │
          ┌──→ │  Dd1 SENSING       │               │
               │  TRANSFER          │               │
               └─────────┬──────────┘               │
         ST4        ╱────┴────╲                      │
  Dd1>Dth  ┌──────< COMPARE Dd1 >──────┐ Dd1<Dth     │
           │        ╲ WITH Dth╱         │            │
  ┌────────┴───────┐        ┌───────────┴────────┐   │
  │ DECREASE NUMBER│        │ INCREASE NUMBER OF │   │
  │ OF OPENED      │        │ OPENED HOLES 15    │   │
  │ HOLES 15       │        └──────────┬─────────┘   │
  └───────┬────────┘              ST6                 │
      ST5      ╱────────────╲        NO               │
       ┌─────< Dd1 COINCIDES >───────────────────────┘
   ST7   ╲ WITH THRESHOLD╱
          ╲   VALUE      ╱  Dd1=Dth
               │ YES
       ┌───────┴────────┐
  ST8  │ MAINTAIN LIQUID│
       │ CRYSTAL        │
       │ SWITCHING      │
       │ CONTROL        │
       └───────┬────────┘
  ST9     ╱────┴────╲       NO
      ┌──< FINISHED ? >─────────┐
          ╲─────────╱
               │ YES
          ┌────┴─────┐
          │   END    │
          └──────────┘
```

**【FIG. 10】**

【FIG. 11】　　　　11/21

<u>300</u>

START

ST11 — SETTING ?　　NO

YES

ST12 — ENERGIZE LIQUID CRYSTAL DISPLAY ELEMENT (CROSS DISPLAY)

ST13 — FINISHED?　　NO

YES

END

【FIG. 12】　　　　　　　　12/21

【FIG. 13】                    13/21

400

START

ST21 — SETTING ? — NO

YES

ST22 — ENERGIZE SURFACE-EMITTING ELEMENTS IN LIGHT-SHIELDING REGION

ST23 — Dc1 RECEPTION TRANSFER

ST24 — CONTROL DISPLAY OF SURFACE-EMITTING ELEMENT

ST25 — FINISHED ? — NO

YES

END

【FIG. 14】 14/21

【FIG. 15】 15/21

【FIG. 16】 16/21

500

```
                    ┌──────────────┐
                    │    START     │
                    └──────┬───────┘
                           │
                           ▼
ST31                  ╱─────────╲         NO
              ╱──────────────────────────╲──────────────┐
              ╲      SETTING ?      ╱                    │
                   ╲─────────╱                           │
                        │ YES                            │
                        ▼                                │
ST32        ┌────────────────────────────────┐          │
            │ ENERGIZE LIQUID CRYSTAL DISPLAY │          │
            │  ELEMENT IN TRANSPARENT REGION  │          │
            └────────────────┬───────────────┘          │
                             │                           │
                             ▼                           │
ST33        ┌──────────────────────┐                     │
            │   Dc2 RECEPTION       │                     │
            │   TRANSFER            │                     │
            └──────────┬───────────┘                     │
                       │                                 │
                       ▼                                 │
ST34        ┌──────────────────────┐                     │
            │   CONTROL LIQUID      │                     │
            │   CRYSTAL DISPLAY     │                     │
            └──────────┬───────────┘                     │
                       │                                 │
                       ▼                                 │
ST35               ╱─────────╲         NO                │
                  ╱───────────╲────────────────────────┘
                  ╲ FINISHED ? ╱
                   ╲─────────╱
                        │ YES
                        ▼
                 ┌──────────────┐
                 │     END      │
                 └──────────────┘
```

**【FIG. 17A】**          17/21

【FIG. 17B】 18/21

**【FIG. 18】** 19/21

600

START

ST41 — SETTING ? — NO

YES

ST42 — ENERGIZE INTRAOCULAR PRESSURE SENSING ELEMENT

ST43 — Dd2 SENSING TRANSFER

ST44 — CONTROL INFORMATION RECORDING/DRUG SUSTAINED RELEASE

ST45 — FINISHED ? — NO

YES

END

【FIG. 19】　　　　　20/21

**【FIG. 20】** 21/21

700

```
                    ┌─────────────┐
                    │    START    │
                    └──────┬──────┘
                           │ ◄──────────────────────────┐
                           ▼                             │
ST51 ──────         ╱◇╲                                  │
                 ╱       ╲       NO                      │
                ◇ SETTING ? ◇ ──────────────────────────┤
                 ╲       ╱                               │
                    ╲◇╱                                  │
                     │ YES                               │
                     ▼                                   │
ST52 ──────  ┌───────────────────────┐                  │
             │ ENERGIZE IMAGING       │                  │
             │ ELEMENT                │                  │
             │ (IMAGE ACQUISITION)    │                  │
             └───────────┬───────────┘                  │
                         ▼                               │
ST53 ──────        ╱◇╲                                   │
                ╱       ╲      NO                        │
               ◇ FINISHED ? ◇ ──────────────────────────┘
                ╲       ╱
                   ╲◇╱
                    │ YES
                    ▼
             ┌─────────────┐
             │     END     │
             └─────────────┘
```

## INTERNATIONAL SEARCH REPORT

| International application No. |
| --- |
| PCT/JP2017/040931 |

| A. CLASSIFICATION OF SUBJECT MATTER |
| --- |
| Int.Cl. G02C7/04(2006.01)i, H04B1/3827(2015.01)i |

According to International Patent Classification (IPC) or to both national classification and IPC

| B. FIELDS SEARCHED |
| --- |
| Minimum documentation searched (classification system followed by classification symbols) |
| Int.Cl. G02C7/04, H04B1/3827 |

| Documentation searched other than minimum documentation to the extent that such documents are included in the fields searched |
| --- |
| Published examined utility model applications of Japan          1922–1996 |
| Published unexamined utility model applications of Japan        1971–2018 |
| Registered utility model specifications of Japan                1996–2018 |
| Published registered utility model applications of Japan        1994–2018 |

| Electronic data base consulted during the international search (name of data base and, where practicable, search terms used) |
| --- |
| |

| C. DOCUMENTS CONSIDERED TO BE RELEVANT |
| --- |

| Category* | Citation of document, with indication, where appropriate, of the relevant passages | Relevant to claim No. |
| --- | --- | --- |
| Y | WO 2011/048953 A1 (UNIVERSAL VIEW CO., LTD.) 28 April 2011, paragraphs [0013]-[0036], fig. 1-3 & US 2012/0200821 A1, paragraphs [0023]-[0046], fig. 1-3 | 1-23 |
| Y | JP 2016-2353 A (SONY CORP.) 12 January 2016, paragraphs [0036]-[0106], [0138]-[0148], [0272]-[0293], fig. 1-6, 16 & US 2017/0119311 A1, paragraphs [0043]-[0115], [0149]-[0160], [0288]-[0310], fig. 1-6, 16 | 1-23 |

☒ Further documents are listed in the continuation of Box C.  ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 19 January 2018 (19.01.2018) | 30 January 2018 (30.01.2018) |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

International application No.

PCT/JP2017/040931

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2014/178221 A1 (SONY CORP.) 06 November 2014, paragraphs [0020]-[0064], fig. 1-5 & US 2016/0299357 A1, paragraphs [0042]-[0097], fig. 1-5 | 1-23 |
| Y | JP 2013-501598 A (ACUFOCUS, INC.) 17 January 2013, paragraph [0174], fig. 45 & WO 2011/020078 A1, paragraph [0286], fig. 45 | 7, 23 |
| Y | JP 2013-218326 A (JOHNSON & JOHNSON VISION CARE INC.) 24 October 2013, paragraph [0050], fig. 5 & US 2013/0258275 A1, paragraph [0062], fig. 5 | 17, 23 |
| Y | JP 2015-515115 A (JOHNSON & JOHNSON VISION CARE INC.) 21 May 2015, paragraph [0068], fig. 3 & US 2012/0162600 A1, paragraph [0129], fig. 9 | 21, 23 |
| Y | JP 2016-534767 A (VERILY LIFE SCIENCES LLC) 10 November 2016, fig. 7A-7E & US 8922366 B1, fig. 7A-7E | 23 |
| A | JP 2014-160258 A (KONNO, Hikoyuki) 04 September 2014, paragraphs [0020]-[0037], fig. 1-8 (Family: none) | 1-23 |
| A | JP 2015-18179 A (UNIVERSAL VIEW CO., LTD.) 29 January 2015, paragraphs [0029]-[0071], fig. 1-6 (Family: none) | 1-23 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4828000 B
- JP 2013122591 A
- JP 4752309 B
- US 8608310 B
- US 9158133 B
- JP 5992424 B
- JP 5244092 B
- US 20160097940 A